(19) Europäisches Patentamt
European Patent Office
Office européen des brevets

(11) **EP 1 941 912 A1**

(12) **EUROPEAN PATENT APPLICATION**
published in accordance with Art. 153(4) EPC

(43) Date of publication:
**09.07.2008 Bulletin 2008/28**

(51) Int Cl.:
*A61L 2/02* *(2006.01)*   *A61L 2/10* *(2006.01)*
*A61L 2/14* *(2006.01)*

(21) Application number: **06822108.4**

(22) Date of filing: **24.10.2006**

(86) International application number:
**PCT/JP2006/321128**

(87) International publication number:
**WO 2007/049595 (03.05.2007 Gazette 2007/18)**

(84) Designated Contracting States:
**AT BE BG CH CY CZ DE DK EE ES FI FR GB GR HU IE IS IT LI LT LU LV MC NL PL PT RO SE SI SK TR**

(30) Priority: **25.10.2005 JP 2005310196**
**01.03.2006 JP 2006054500**
**23.03.2006 JP 2006080651**

(71) Applicant: **NGK INSULATORS, LTD.**
**Nagoya-shi, Aichi 467-8530 (JP)**

(72) Inventors:
• **SHIMIZU, Naohiro**
**Nagoya-shi**
**Aichi 467-8530 (JP)**

• **WAKITA, Masahiro**
**Nagoya-shi**
**Aichi 467-8530 (JP)**
• **IMANISHI, Yuichiro**
**Nagoya-shi**
**Aichi 467-8530 (JP)**

(74) Representative: **Paget, Hugh Charles Edward et al**
**Mewburn Ellis LLP**
**York House**
**23 Kingsway**
**London WC2B 6HP (GB)**

(54) **STERILIZING DEVICE**

(57)    An aseptization apparatus 1 includes a sealed container 11 forming an aseptization space 191, a nitrogen gas supplying system 12 for converting atmosphere of the aseptization space 191 into nitrogen atmosphere, an electrode pair 13 disposed in the aseptization space 191, a pulse power supply 14 for repeatedly applying an electric pulse to the electrode pair 13, and a mirror 15 for returning a short-wavelength ultraviolet ray going from inside of the aseptization space 191 to outside to inside of the aseptization space 191. In a state where an asep-tization object substance ST1 is present in a plasma generation region 192 between the electrode pair 13, the aseptization apparatus 1 causes a pulse electric field generated by electric pulse application to the electrode pair 13, a nitrogen radical 195 contained in plasma generated in nitrogen atmosphere resulting from fine stream-er discharge, and a short-wavelength ultraviolet ray 196 emitted by nitrogen atmosphere resulting from fine streamer discharge to act on bacteria for aseptization of the aseptization object substance ST1.

F I G. 5

EP 1 941 912 A1

**Description**

TECHNICAL FIELD

The present invention relates to a sterilization/aseptization apparatus for sterilizing or aseptizing a target substance.

BACKGROUND ART

Conventionally, ethylene oxide gas method, hydrogen peroxide gas plasma method, gamma-ray irradiation method, electron beam irradiation method, heating method, ultraviolet ray method, and the like have been used for sterilization and aseptization purposes.

Among afore-mentioned methods, ethylene oxide gas method has such a problem that ethylene oxide gas used for sterilization and aseptization has toxic consequences. In addition, ethylene oxide gas is suspected for its carcinogenicity, and it is predicted that regulations imposed on ethylene oxide gas will become stricter in the future.

In the meantime, with hydrogen peroxide gas plasma method, which has higher safety than ethylene oxide gas method, there is a restriction that application to such a case where a steric substance to be subjected to sterilization and aseptization is made of cellulose is not possible, and this method is also not said to be a versatile method.

Further, there is such a problem with gamma-ray irradiation method that management of gamma-ray source such as cobalt 60 is troublesome.

For this reason, electron beam irradiation method, heating method, and ultraviolet ray method are employed in many cases from viewpoints of higher safety and higher versatility.

DISCLOSURE OF THE INVENTION

However, electron beam irradiation method, heating method, and ultraviolet ray method have such problems that a large quantity of energy is consumed for sterilization or aseptization, and apparatuses used for sterilization and aseptization tend to become large-scaled.

The present invention is developed to resolve these problems and an object of the present invention is to realize a simplified sterilization/aseptization apparatus capable of performing sterilization or aseptization with a small amount of energy.

In order to attain above-mentioned object, a sterilization/aseptization apparatus according to a first aspect is a sterilization/aseptization apparatus for sterilizing or aseptizing a target substance, including an atmosphere control means for converting atmosphere of a space where sterilization or aseptization is performed into nitrogen atmosphere, an electrode pair disposed in said space, a pulse power supply for repeatedly applying an electric pulse, which induces a fine streamer discharge without inducing arc discharge, to said electrode pair, a reflection member for returning short-wavelength ultraviolet ray going from inside of said space to outside to inside of said space, wherein said object substance is sterilized or aseptized by causing a pulse electric field generated by application of the electric pulse to said electrode pair, nitrogen radical contained in plasma generated in the nitrogen atmosphere resulting from the fine streamer discharge, and short-wavelength ultraviolet ray emitted by the nitrogen atmosphere resulting from the fine streamer discharge to act on bacteria.

With this feature, in addition to that cell membrane of bacteria is destroyed by the pulse electric field and DNA of bacteria is destroyed by the nitrogen radical, short-wavelength ultraviolet ray having high bactericidal effect can be caused to act effectively on bacteria, and therefore, sterilization or aseptization can be carried out with a small amount of energy.

A sterilization/aseptization apparatus according to a second aspect is the sterilization/aseptization apparatus according to the first aspect, wherein pulse width of said electric pulse is from 50 to 300 ns in terms of half bandwidth.

A sterilization/aseptization apparatus according to a third aspect is the sterilization/aseptization apparatus according to the first aspect or the second aspect, wherein said atmosphere control means generates a nitrogen gas flow which is parallel to said pulse electric field.

This allows improvement of uniformity of aseptization, and generation of ozone can be prevented even if a space where aseptization takes place is not sealed completely.

A sterilization/aseptization apparatus according to a fourth aspect is the sterilization/aseptization apparatus according to any one of the first aspect through the third aspect, wherein said object substance is a sheet, said atmosphere control means generates a nitrogen gas flow vertical to said sheet by injecting nitrogen gas towards said sheet, said electrode pairs are opposed each other across said sheet, and said sheet is sterilized or aseptized by causing said pulse electric field, said nitrogen radical, and said short-wavelength ultraviolet ray to act on bacteria adhered to said sheet.

With this feature, both faces of the sheet can be sterilized or aseptized with a small amount of energy. Further, since chemical species caused by reaction with nitrogen radical is removed from vicinity of the sheet by nitrogen gas flow of blow over of nitrogen gas flow going towards the sheet, sterilization or aseptization can be performed in stable fashion at higher efficiency.

A sterilization/aseptization apparatus according to a fifth aspect is the sterilization/aseptization apparatus according to the fourth aspect, further including a carrying means for causing said sheet to travel along with a predetermined carrying route.

A sterilization/aseptization apparatus according to a sixth aspect is the sterilization/aseptization apparatus according to any one of the first aspect through the third aspect, wherein said object substance is a steric substance, which apparatus further includes a pressure reducing means for pressure reducing said space, and said sheet is sterilized or aseptized by causing said pulse electric field, said nitrogen radical, and said short-wavelength ultraviolet ray to act on bacteria adhered to said sheet.

With this feature, fine streamer discharge is induced in reduced pressured nitrogen atmosphere, and therefore, discharge distance can be lengthened and at the same time, lifetime of nitrogen radical can be lengthened, thereby realizing a sterilization/aseptization apparatus suited for effective sterilization or aseptization of a steric substance.

A sterilization/aseptization apparatus according to a seventh aspect is the sterilization/aseptization apparatus according to the sixth aspect, further including a rotary mechanism for causing said steric substance to rotate around a rotating shaft being vertical to said pulse electric field.

This allows that entire steric substance is faced sequentially towards anode side which exhibits higher bactericidal effect, and therefore, entire steric substance can be sterilized or aseptized uniformly.

A sterilization/aseptization apparatus according to an eighth aspect is the sterilization/aseptization apparatus according to the sixth aspect, further including a rotary mechanism for causing anode and cathode of said electrode pair to rotate around a rotating shaft being vertical to said pulse electric field and to revolve around said steric substance with the same cycle.

This allows that entire steric substance is faced sequentially towards anode side which exhibits higher bactericidal effect, and therefore, entire steric substance can be sterilized or aseptized uniformly.

A sterilization/aseptization apparatus according to a ninth aspect is the sterilization/aseptization apparatus according to any one of the sixth aspect through the eighth aspect, wherein a distance between said electrode pair is not less than 1.2 times of length in the longest direction of said object substance.

This allows that the object substance can be exposed effectively to nitrogen radical without damaging the object substance even if arc discharge occurs accidentally.

A sterilization/aseptization apparatus according to a tenth aspect is the sterilization/aseptization apparatus according to any one of the sixth aspect through the ninth aspect, further including a holding means for holding an aseptization object inside said space, wherein at least a part of said holding means is in mesh shape or comb teeth shape.

This allows that, since short-wavelength ultraviolet ray is not blocked by the holding means, short-wavelength ultraviolet ray can be irradiated effectively to an object substance.

A sterilization/aseptization apparatus according to an eleventh aspect is the sterilization/aseptization apparatus according to the tenth aspect, wherein material of said holding means is iron alloy or tungsten alloy. [0027]This allows improvement of durability of the holding means.

A sterilization/aseptization apparatus according to a twelfth aspect is the sterilization/aseptization apparatus according to any one of the sixth aspect through the eleventh aspect, further including a turbulence generation means for generating a turbulence in the nitrogen atmosphere and exposing said object substance to the turbulence.

This allows that, since entire object substance can be exposed to nitrogen radical, entire object substance can be sterilized or aseptized uniformly.

A sterilization/aseptization apparatus according to a thirteenth aspect is the sterilization/aseptization apparatus according to any one of the first aspect through the twelfth aspect, wherein said reflection member has a reflection coefficient of said short-wavelength ultraviolet ray not less than 30%.

This allows that short-wavelength ultraviolet ray can be irradiated effectively to an object substance.

A sterilization/aseptization apparatus according to a fourteenth aspect is the sterilization/aseptization apparatus according to the thirteenth aspect, wherein said reflection member reflects said short-wavelength ultraviolet ray by an aluminum film.

This allows that, since reflection coefficient of short-wavelength ultraviolet ray becomes high, short-wavelength ultraviolet ray can be irradiated effectively to an object substance.

A sterilization/aseptization apparatus according to a fifteenth aspect is the sterilization/aseptization apparatus according to the fourteenth aspect, wherein said aluminum film is covered with silicon oxide film or magnesium fluoride film.

This allows, since the aluminum film is protected, improvement of durability of the reflection member.

A sterilization/aseptization apparatus according to a sixteenth aspect is the sterilization/aseptization apparatus according to any one of the first aspect through the fifteenth aspect, further including a temperature regulating means for regulating a temperature of said nitrogen atmosphere.

## EP 1 941 912 A1

This allows that, since it is possible to attain nitrogen atmosphere temperature suited for sterilization or aseptization, an object substance can be sterilized or aseptized effectively.

A sterilization/aseptization apparatus according to a seventeenth aspect is the sterilization/aseptization apparatus according to any one of the first aspect through the sixteenth aspect, wherein at least one of anode and cathode of said electrode pair is an electrode plate in mesh shape or comb teeth shape.

This allows that, since short-wavelength ultraviolet ray is not blocked by anode or cathode, short-wavelength ultraviolet ray can be irradiated effectively to an object substance.

A sterilization/aseptization apparatus according to an eighteenth aspect is the sterilization/aseptization apparatus according to any one of the first aspect through the seventeenth aspect, wherein material of at least one of anode and cathode of said electrode pair is nickel alloy or iron alloy.

This allows improvement of durability of the electrode pair.

A sterilization/aseptization apparatus according to a nineteenth aspect is the sterilization/aseptization apparatus according to any one of the first aspect through the eighteenth aspect, wherein a part of or whole anode and cathode of said electrode pair are covered with an insulating material.

This allows improvement of durability of the electrode pair.

A sterilization/aseptization apparatus according to a twentieth aspect is the sterilization/aseptization apparatus according to any one of the first aspect through the twentieth aspect, wherein the anode of said electrode pair is a cylindrical-shape electrode having a diameter not less than 0.3 mm and not more than 3.0 mm.

A sterilization/aseptization apparatus according to a twenty-first aspect is the sterilization/aseptization apparatus according to any one of the first aspect through the twentieth aspect, wherein the cathode of said electrode pair includes a quartz glass plate, and an aluminum film being formed onto one of principal surfaces of said quartz glass plate, and another principal surface of said quartz glass plate is faced towards said object substance.

This allows, since the quartz glass plate functions as a dielectric body barrier, time before electric current is discontinued, when an electric pulse is applied, is lengthened, and input electric power can be increased. This results in increase in the nitrogen atmosphere temperature up to a level suited for sterilization or aseptization, thereby improving sterilization or aseptization efficiency. Further, since it is possible to invert a flowing electric current midway in response to application of the electric pulse, charge-up of the sheet can be prevented and unevenness of glow discharge can be prevented. Further, since the cathode reflects short-wavelength ultraviolet ray, the cathode is able to serve also as the reflection member.

A sterilization/aseptization apparatus according to a twenty-second aspect is the sterilization/aseptization apparatus according to any one of the first aspect through the twenty-first aspect, wherein said atmosphere control means includes a plate-shape member to which a penetration hole penetrating through both principal surfaces is formed, and nitrogen gas is injected through said penetration hole.

A sterilization/aseptization apparatus according to a twenty-third aspect is the sterilization/aseptization apparatus according to the twenty-second aspect, wherein a plurality of first grooves extending in a first direction are excavated to one principal surface of said plate-shape member and at the same time, a plurality of second groves extending in a second direction, which is different from said first direction, are excavated to other principal surface of said plate-shape member, while an intersection of said first grooves and said second grooves serves as said penetration hole.

This allows manufacturing of plate-shape members in short time at inexpensive costs.

A sterilization/aseptization apparatus according to a twenty-fourth aspect is the sterilization/aseptization apparatus according to the twenty-second aspect or the twenty-third aspect, wherein said atmosphere control means further includes a mesh being laminated onto one principal surface of said plate-shape member, and injects nitrogen gas sequentially passing through openings of said mesh and said penetration hole of said plate-shape member.

This allows uniform injection of nitrogen gas from a number of penetrating holes.

A sterilization/aseptization apparatus according to a twenty-fifth aspect is the sterilization/aseptization apparatus according to the twenty-second aspect or the twenty-third aspect, wherein said atmosphere control means further includes a ceramic madreporite on one principal surface of said plate-shape member, and injects nitrogen gas sequentially passing through openings of said ceramic madreporite and said penetration hole of said plate-shape member.

This allows that, since injection of nitrogen gas from a number of penetration holes can be made uniform, fluctuation of the amount of nitrogen gas injection in the surface can be maintained at not more than 10%.

A sterilization/aseptization apparatus according to a twenty-sixth aspect is the sterilization/aseptization apparatus according to any one of the first aspect through the twenty-fifth aspect, further including a mechanism for moving or rotating the cathode of said electrode pair in vertical direction with regard to said pulse electric field.

BRIEF DESCRIPTION OF THE DRAWINGS

**4**

EP 1 941 912 A1

FIG. 1 is a drawing schematically showing a bacterium 80 exposed to electric field E and distribution of potential $\phi$ in the direction of electric field E.

FIG. 2 is a schematic drawing showing a state where a crack is caused to a cell membrane 81.

FIG. 3 is a drawing showing results of experiments for checking changes in absorption coefficient (vertical-axis) of ultraviolet ray due to DNA with regard to wavelength (horizontal-axis).

FIG. 4 is a drawing showing results of experiments for checking changes in bactericidal effect (vertical-axis) of ultraviolet ray with regard to wavelength (horizontal-axis).

FIG. 5 is a drawing schematically showing rough waveforms of electric pulse to be applied to an electrode pair 73 and discharge induced by the electric pulse.

FIG. 6 is a cross-sectional view schematically showing an example of the configuration of an aseptization apparatus 1 according to a first embodiment of the present invention.

FIG. 7 is a circuit diagram of an IES circuit 140.

FIG. 8 is a graph showing wavelength dependence of reflection coefficient of various metallic films.

FIG. 9 is a cross-sectional view schematically showing an example of the configuration of the aseptization apparatus 1 according to a variant of the first embodiment.

FIG. 10 is a cross-sectional view schematically showing an example of the configuration of the aseptization apparatus 1 according to a variant of the first embodiment.

FIG. 11 is a cross-sectional view schematically showing an example of the configuration of an aseptization apparatus 2 according to a second embodiment of the present invention.

FIG. 12 is a cross-sectional view showing the cross-sectional structure of a cathode 232 which also serves as a mirror 25.

FIG. 13 is a perspective view showing whole configuration of an aseptization apparatus 3 according to a third embodiment of the present invention.

FIG. 14 is a cross-sectional view showing the configuration of a reactor 31.

FIG. 15 is a cross-sectional view showing the configuration of the reactor 31.

FIG. 16 is a perspective view showing the configuration of a hole drilling mirror 351.

FIG. 17 is a drawing showing rough waveform of electric pulse to be applied to an electrode pair 33.

FIG. 18 is a drawing showing a state of space SP3 when electric pulse is applied to the electrode pair 33.

FIG. 19 is a drawing showing a state of space SP3 when electric pulse is applied to the electrode pair 33.

FIG. 20 is a drawing showing a relationship between flow rate of nitrogen gas and aseptization efficiency.

FIG. 21 is a cross-sectional view showing another example of internal configuration of the reactor 31.

FIG. 22 is a perspective view showing rough configuration of whole aseptization apparatus 4 according to a fourth embodiment of the present invention.

FIG. 23 is a cross-sectional view showing the configuration of a reactor 41.

FIG. 24 is a drawing showing a state of aseptization space SP3 when electric pulse is applied to an electrode pair 43.

FIG. 25 is a drawing showing a state of aseptization space SP3 when electric pulse is applied to the electrode pair 43.

FIG. 26 is a drawing showing the configuration of the reactor 41 according to a variant of the fourth embodiment.

FIG. 27 is a perspective view showing rough configuration of whole aseptization apparatus 5 according to a fifth embodiment of the present invention.

FIG. 28 is a cross-sectional view showing the configuration of a reactor 51.

FIG. 29 is a cross-sectional view showing another example of the configuration of the reactor 51.

FIG. 30 is a cross-sectional view showing another example of the configuration of the reactor 51.

FIG. 31 is a drawing showing plasma PR 5 generation state. [0054]FIG. 32 is a drawing showing rough waveform of electric pulse. [0054]FIG. 33 is a drawing for explanation of operations of the aseptization apparatus 5.

FIG. 34 is a drawing showing results of experimental assessment of success rate of bacterial death performed using 100 pieces of biological indicators (assessment 1), and results of experimental assessment of success rate of destroying DNA of bacteria in the biological indicators (assessment 2).

BEST MODE FOR CARRYING OUT THE INVENTION

<1 Principle of aseptization>

With the aseptization apparatus of the present invention, aseptization is carried out with a small amount of energy by causing pulse electric field, nitrogen radical, short-wavelength ultraviolet ray including wavelength region where wavelength is not less than 100 nm and not more than 280 nm (referred to as "far-ultraviolet ray" or "UV-C") to act on bacteria compositely. For the sake of better understanding of the principle of aseptization used in the aseptization apparatus of the present invention, the following description then explains sequentially actions which pulse electric field, nitrogen radical, and short-wavelength ultraviolet ray exert on bacteria.

Although the difference between "sterilization" and "aseptization" is whether or not bacteria are killed completely, it is apparent that the technology of "aseptization" by which bacteria are killed completely can be applied to "sterilization" by which bacteria are not killed completely, and therefore, the term "aseptization apparatus" as used hereunder should be considered to be substantially synonymous with "sterilization/aseptization apparatus" which sterilizes or aseptizes an object substance.

<1.1 Actions given to bacteria by pulse electric field>

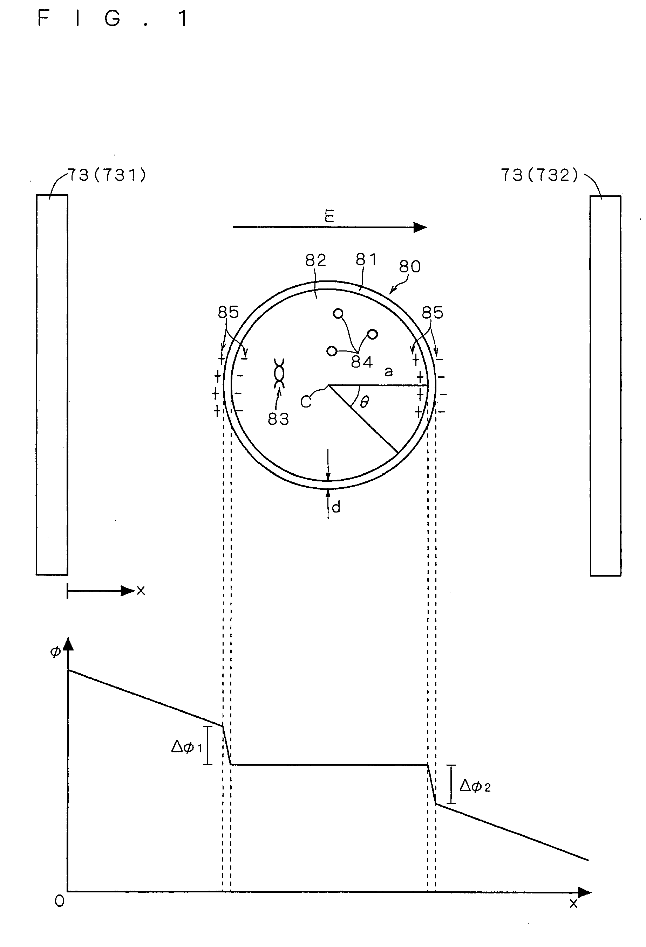

FIG. 1 is a drawing schematically showing a bacterium 80 exposed to electric field E and distribution of potential $\phi$ in the direction of electric field E.

As shown in FIG. 1, the bacterium (cell) 80 has such a structure that a cell cytoplasm 82 is covered with the cell membrane 81 having phospholipid dual layer structure, and holds a DNA (deoxyribo nucleic acid) 83 and a toxin 84. It may be considered that the bacterium 80 has such a structure that the cell cytoplasm 82, which is a conductor, is covered with the cell membrane 81, which is dielectric body.

When the bacterium 80 is exposed to electric field E, a polarized charge 85 is induced on external surface and internal surface of the cell membrane 81, and an inner electric field $E_m$ having higher intensity than an outer electric field $E_{ex}$ is generated in the cell membrane 81. Here, suppose that the bacterium 80 is spherical, then ratio of internal electric field $E_m$ to external electric field $E_{ex}$, i.e., electric field increasing rate $\varepsilon$ is expressed by Equation 1 using radius a of the cell, thickness d of the cell membrane 81, and angle $\theta$ formed between direction of electric field E and direction from cell center C towards a portion of attention focusing.

[Mathematical expression 1]

$$\varepsilon = \frac{E_m}{E_{ex}} = \frac{1.5\, a \cos\theta}{d} \qquad \text{(Equation 1)}$$

By this electric field increasing effect, when the bacterium 80 is exposed to electric field E, big potential differences $\Delta\phi 1$ and $\Delta\phi 2$ are caused between external surface and internal surface of the cell membrane 81 of the dielectric body, although no potential difference is caused at cell cytoplasm 82 portion of the dielectric body.

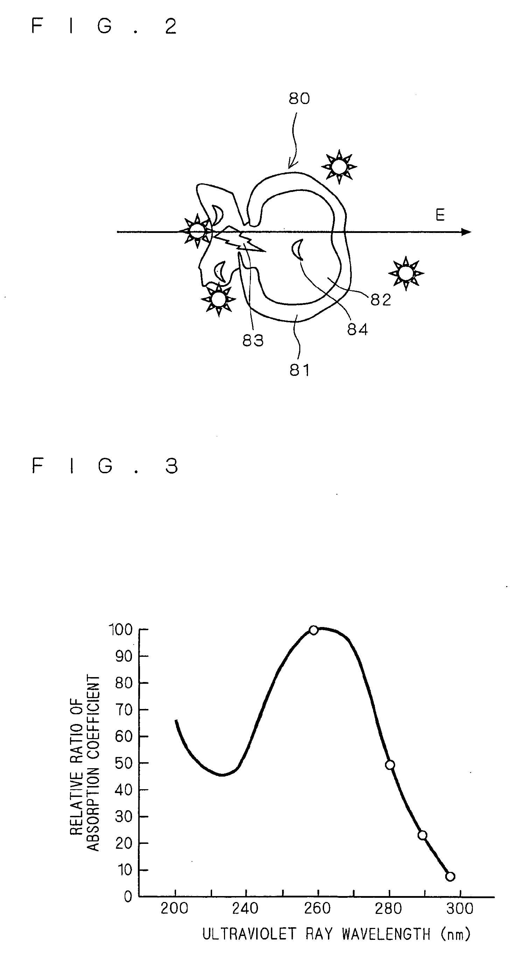

Therefore, if an electric field pulse with fast rising is caused to act on the bacterium 80, and potential differences $\Delta\phi 1$ and $\Delta\phi 2$ between external surface and internal surface of the cell membrane 81 are increased rapidly, the cell membrane 81 of the bacteria 80 will be destroyed without a chance for repairing the cell, resulting in a state as shown in schematic diagram FIG. 2 where a crack is caused to the cell membrane 81. In other words, when an electric pulse with fast rising, i.e., electric pulse of voltage V with higher increasing rate with time (dV/dt) is applied to the electrode pair 73, while an aseptization object substance to which the bacterium 80 is adhered is present between the electrode pair 73 configured of an anode 731 and a cathode 732, it is possible to kill the bacterium 80 and to aseptize the aseptization object substance. Further, application of an electric pulse with fast rising to the electrode pair 73 makes it possible to form a stable plasma region over a distance between the anode 731 and the cathode 732 more than several millimeters even in the atmosphere (typically, 5 to 30 mm under normal pressure). Further there is an advantage that nitrogen gas can be introduced uniformly parallel to electric field E.

<1.2 Actions given to bacteria by nitrogen radical>

With the aseptization apparatus of the present invention, nitrogen radical with extremely high activity is caused to act on the bacterium 80 to destroy the DNA 83 and the toxin 84 which cannot be destroyed by afore-mentioned pulse electric field.

More specifically, with the aseptization apparatus of the present invention, nitrogen radical contained in a plasma generated in nitrogen atmosphere is caused to act on the bacterium 80. The plasma can be generated by applying an electric pulse with fast rising to the electrode pair 73 disposed in nitrogen atmosphere to induce fine streamer discharge. That is, the plasma can be generated by rapidly increasing the voltage to be applied to the electrode pair 73 disposed in nitrogen atmosphere so as to allow growth of electronic avalanche moving from the cathode 732 to the anode 731.

The reason why nitrogen radical is selected as an active species to be utilized for aseptization, i.e., the reason for generation of plasma in nitrogen atmosphere, is that activity of nitrogen radical is markedly higher than other active species, e.g., oxygen radical. This is supported by the fact that dissociation energy of ozone molecule is 1.05 eV while dissociation energy of nitrogen molecule is 9.76 eV. Furthermore, the fact that lifetime of nitrogen radical is long, for example, lifetime of biradical of triplet nitrogen ($^3\Sigma u$) reaches 10 millisecond, is one of reasons to explain why nitrogen radical is selected as the active species utilized for aseptization.

In addition, ease of availability of nitrogen gas at low price and ease of handing thereof also contribute to the reason why nitrogen radical is selected as the active species utilized for aseptization.

<1.3 Actions given to bacteria by short-wavelength ultraviolet ray>

With the aseptization apparatus of the present invention, short-wavelength ultraviolet ray with wavelength of about 250 nm, which is emitted by nitrogen atmosphere resulting from afore-mentioned fine streamer, is irradiated to an aseptization object substance.

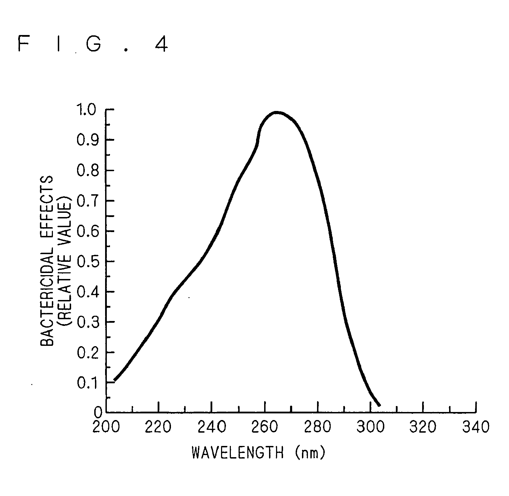

As show in FIG.3 and FIG.4, utilization of short-wavelength ultraviolet ray with wavelength of about 250 nm is attributable to that considerations are given to that absorption coefficient of ultraviolet ray by DNA becomes higher (see FIG. 3) around wavelength of 250 nm and to that bactericidal effect of ultraviolet ray is increased (see FIG. 4). Meanwhile, FIG. 3 is a graph showing results of experiments for checking changes in the absorption coefficient (vertical-axis) of ultraviolet ray due to DNA with regard to wavelength (horizontal-axis), and FIG. 4 is a graph showing results of experiments for checking changes in bactericidal effect (vertical-axis) of ultraviolet ray to wavelength (horizontal-axis).

Meanwhile, such a problem arises that when atmosphere other than nitrogen atmosphere, e.g., oxygen atmosphere, is selected, wavelength of ultraviolet ray emitted by oxygen atmosphere reaches about 400 nm, thereby loosing bactericidal effects substantially (see FIG. 4). In addition, there is another problem that oxygen atmosphere attenuates short-wavelength ultraviolet ray which has bactericidal effects.

<1.4 Fine streamer discharge>

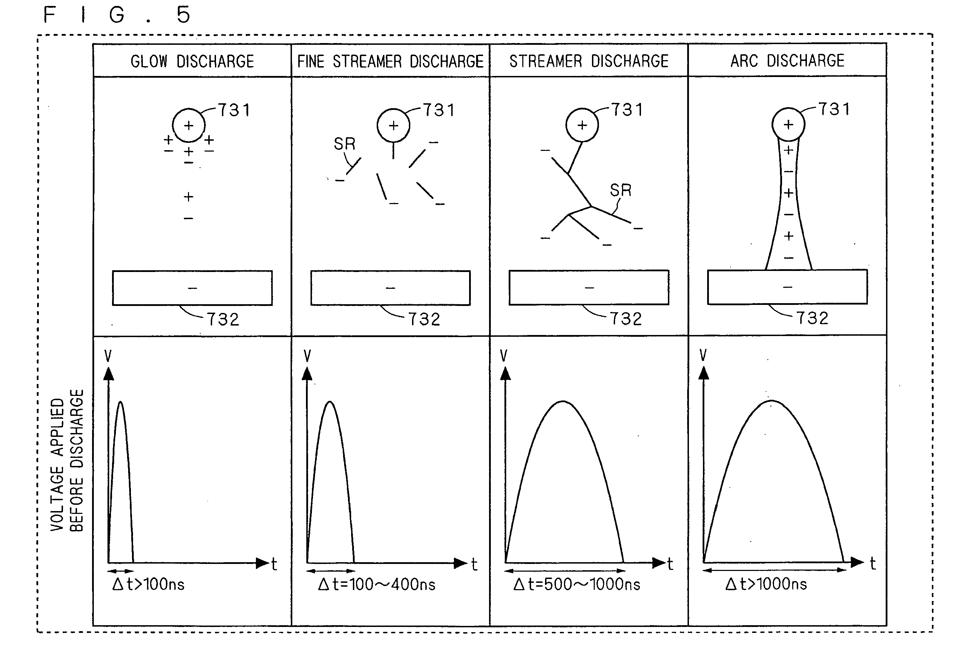

FIG. 5 is a drawing schematically showing rough waveforms of electric pulse to be applied to the electrode pair 73 and discharge induced by the electric pulse. In FIG. 5, rough waveform of the voltage of electric pulse before discharge is illustrated by a graph showing changes in voltage V (horizontal-axis) to time t (vertical-axis).

As shown in FIG. 5, when pulse width $\Delta t$ of the electric pulse reaches around 100 ns (typically, 50 to 300 ns in terms of half bandwidth), secondary electron emitted at collision of positive ion with the cathode 732 ionizes nitrogen molecule thereby inducing glow discharge which generates new positive ions.

In the meantime, when increasing rate of voltage V with time (dV/dt) at rising of the electric pulse is around 30 to 50 kV/$\mu$s, and when pulse width $\Delta t$ reaches around 100 ns, growth of streamer SR extending from the anode 731 to the cathode 732 starts. When pulse width $\Delta t$ is around 100 to 400 ns, growth of streamer SR is terminated at initial stage where short streamer SR is scattered between the anode 731 and the cathode 732. Meanwhile, when pulse width $\Delta t$ is around 500 to 1000 ns, growth of the streamer SR becomes substantial, and such a state is obtained where branched longer streamer SR is present between an anode 72 and a cathode 70. With the aseptization apparatus of the present invention, it is preferable to use fine streamer discharge which stops discharge at initial stage of growth of streamer SR so that the anode 72 and the cathode 70 may not become conductive due to acceleration of the growth of the streamer SR. This is because use of fine streamer discharge excellent in uniformity of discharge allows uniform aseptization of the aseptization object substance.

Further, when pulse width $\triangle t$ reaches 1000 ns, local current crowding occurs and arc discharge is eventually induced.

In the above-shown explanation, term "around" is used for pulse width $\triangle t$ and increasing rate of voltage V with time (dV/dt) at rising, and this is because they change depending on specific configuration of the aseptization apparatus such as interval of the electrode pair 73, construction of the anode 731 and the cathode 732, pressure of nitrogen atmosphere, and the like. Therefore, whether or not fine streamer discharge is made available should be judged by observation of actual discharge as well as pulse width $\triangle t$ and increasing rate of voltage V with time (dV/dt) at rising.

<2. Preferable embodiment>

The following description explains preferable embodiments of the aseptization apparatus using previously mentioned principle of aseptization.

The aseptization apparatus is capable of killing bacteria adhered onto surface of an aseptization object substance, especially aseptization object substance to which application of the heating method is not possible, and destroying DNA and toxin held by the bacteria. Further, the aseptization apparatus is also capable of inactivating prion, and endotoxin which is toxin produced by gram-negative bacteria.

As aseptization object substance which can be aseptized by the aseptization apparatus, for example, medical devices, packing materials for medical devices and food articles are exemplified. More specifically, the aseptization apparatus is able to perform aseptization of medical devices such as ampule, vial, gasket for vial, syringe, gasket for syringe, cartridge, injection needle, gauze, nonwoven cloth; packing materials for medical devices such as blister pack, film for pillow packaging film; packaging materials for food articles such as bottles, trays. Of course, the aseptization apparatus may perform aseptization of other articles, for example, industrial materials such as semiconductor wafers, liquid crystal glass substrates and ceramics substrates.

<2.1 First embodiment>

<2.1.1 Configuration of aseptization apparatus>

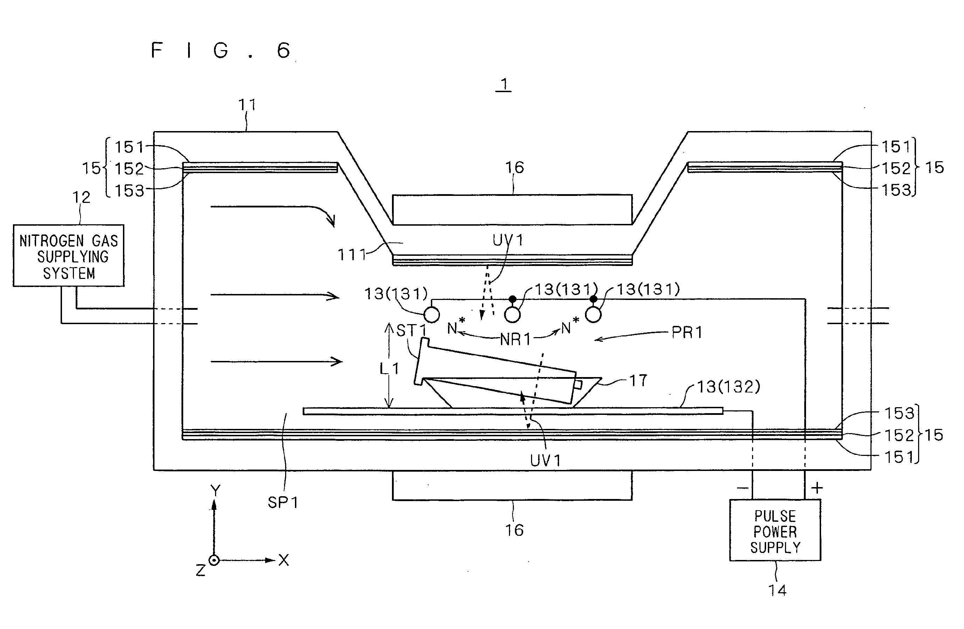

FIG. 6 is a cross-sectional view schematically showing an example of the configuration of the aseptization apparatus 1 according to a first embodiment of the present invention. The aseptization apparatus 1 is configured for aseptization of the surface of aseptization object substance ST1 having steric profile such as syringe. In FIG. 6, for convenience of explanation, X-Y-Z orthogonal coordinate system is defined in which right and left directions are X-axis direction, up and down directions are Y-axis direction, and fore and aft directions are Z-axis direction.
As shown in FIG. 6, the aseptization apparatus 1 includes a hollow sealed container 11 for forming a space (also referred hereinafter to as "aseptization space") SP1 where aseptization is carried out, a nitrogen gas supplying system 12 which converts atmosphere of the aseptization space SP1 to nitrogen atmosphere, an electrode pair 13 disposed in the aseptization space SP1, a pulse power supply 14 for repeatedly applying an electric pulse to the electrode pair 13, a mirror 15 for returning short-wavelength ultraviolet ray UV1 going from inside of the aseptization space SP1 to outside to inside of the aseptization space SP1, a far-infrared ray heater 16 for controlling a temperature of nitrogen atmosphere, and a carrier 17 for holding and carrying the aseptization object substance ST1 inside of the aseptization space SP1.

Sealed container;

The sealed container 11 forms the aseptization space SP1 in cubic shape or cylindrical shape extending in $\pm$ X-direction and introduces nitrogen gas being supplied from the nitrogen gas supplying system 12 to + X-direction.
The sealed container 11 has such a configuration that upper face thereof is immersed towards inside of the aseptization space SP1 in the vicinity of a plasma generation region PR1 sandwiched by the electrode pair 13. That is, in the sealed container 11, area through which nitrogen gas passes is narrowed in the vicinity of the plasma generation region PR1. Therefore, nitrogen gas flowing in + X-direction collides with a protrusion 111 of the sealed container 11, thereby generating turbulence of nitrogen gas in the vicinity of the plasma generation region PR1. In other words, the sealed container 11 includes the protrusion 111 for disturbing nitrogen gas flow and generating turbulence in the nitrogen atmosphere.
In the aseptization apparatus 1, it is possible to expose whole aseptization object substance ST1 to nitrogen radical NR1 contained in plasma generated in the plasma generation region PR1 while a turbulence is generated in the vicinity of the plasma generation region PR1 and the aseptization object substance ST1 is exposed to the turbulence, and thus whole aseptization object substance ST1 can be aseptized uniformly. [0083]Nitrogen gas supplying system;
The nitrogen gas supplying system 12 supplies nitrogen gas to the sealed space SP1 to convert atmosphere of the aseptization space SP1 to nitrogen atmosphere.
The nitrogen gas supplying system 12 controls flow rate of nitrogen gas so that flow velocity of nitrogen gas flowing in a sealed space 191 in + x-direction is not more than 10 m/sec. This is because if flow velocity of nitrogen gas exceeds 10 m/s and becomes remarkably fast, the nitrogen radical NR1 generated in the plasma generation region PR1 is washed away downstream in +X-direction, whereby aseptization effects are reduced.
Further, the nitrogen gas supplying system 12 is supplying nitrogen gas by pressurizing to improve aseptization effects so that the aseptization space SP1 is held at positive pressure, i.e., so that pressure of the aseptization space SP1 is 1.1 to 1.2 times the outside pressure.
Although impurities are sometimes introduced more or less into the nitrogen gas supplied by the nitrogen gas supplying system 12, incorporation of some impurities into the nitrogen gas does not lead to a problem as long as incorporated impurities do not disturb actions of the nitrogen radical NR1 on bacteria and do not attenuate short-wavelength ultraviolet ray UV1. This also applies to second embodiment through fifth embodiment which will be explained below.

Electrode pair;

The anode 131 and the cathode 132 of the electrode pair 13 are disposed in the aseptization space SP1 being separated each other. Distance L1 of the electrode pair 13 is desirably wider than the length of the aseptization object substance ST1 in the longest direction in order to prevent interference with the aseptization object substance ST1 which is present in the plasma generation region PR1 between the electrode pair 13. That is, the distance L1 of the electrode pair 13 is desirably determined so as to have sufficient widening for the plasma generation region PR1 to accommodate the aseptization object substance ST1. Further, if the distance L1 of the electrode pair 13 is widened to more than 1.2 times of the length of the aseptization object substance ST1 in the longest direction, the aseptization object substance ST1 would not be damaged even if arc discharge occurs incidentally between the electrode pair 13, and the aseptization object substance ST1 can be exposed effectively to the nitrogen radical NR1.

Profile of the anode 131 and the cathode 132 of the electrode pair 13 may be, for example, plate-shape or bar-shape. Alternatively, such a configuration may be used that configuration of the anode 131 and the cathode 132 of the electrode pair 13 is cylindrical-shape, while the anode 131 and the cathode 132 are concentric each having different diameter. Here, if the anode 131 and the cathode 132 are designed to be mesh shape or comb teeth shape so that opposite side may be seen through, the short-wavelength ultraviolet ray UV1 is not blocked by the anode 131 and the cathode 132, and therefore, the short-wavelength ultraviolet ray UV1 can be irradiated effectively to the aseptization object substance ST1.

According to the aseptization apparatus 1 shown concretely in FIG. 6, the anode 131 is a cylindrical-shape electrode having diameter of 0.3 to 3.0 mm, length of 10 to 1000 mm, longitudinal direction of which extends in $\pm$ Z-direction, and the cathode 132 is a plate-shape electrode parallel to X-Y plane. Planar pattern of this plate-shape cathode is in mesh-shape so that irradiation of the short-wavelength ultraviolet ray UV1 to the aseptization object substance ST1 is not disturbed.

For materials for the anode 131 and the cathode 132, those composed primarily of metals such as tungsten, molybdenum, manganese, titanium, chrome, zirconium, nickel, silver, iron, copper, platinum and palladium may be used. Of course, use of the anode 131 and the cathode 132 onto which a film composed primarily of these metals is formed using film forming means such as plating is not discouraged. Meanwhile, "metal" as used herein includes an alloy containing two or more kinds of metals.

For materials of the anode 131 and the cathode 132, use of nickel alloy such as INCONEL (registered trademark) and of iron alloy as represented by stainless steel is particularly preferred from viewpoints of improvement of durability.

For the sake of improvement of durability of the anode 131 and the cathode 132, a part of or whole anode 131 and cathode 132 may be covered with insulating materials. For materials of the insulating materials, for example, ceramics such as alumina, magnesia, zirconia, silica, mullite, spinel, Cordierite, aluminum nitride, silicon nitride, titanium-barium oxide, and barium-titanium-zinc oxide may be preferably used. These ceramics can be produced by so-called green sheet lamination method.

Pulse power supply;

Waveform of the electric pulse which is applied to the electrode pair 13 by the pulse power supply 14 is determined so that cell membrane of bacteria is destroyed and at the same time, fire streamer discharge is induced without inducing arc discharge. Typically, waveform of the electric pulse is determined to meet with each of the conditions that increasing rate of voltage V with time (dV/dt) at rising is 50 to 500 kV/$\mu$s, pulse width $\triangle$t is 0.03 to 3.00 $\mu$s, ratio ($V_p$/L1) of peak voltage $V_p$ to distance L1 of the electrode pair 13 is 0.5 to 2.0 kV/cm. After waveform of the electric pulse is thus determined, it becomes possible to induce fine streamer discharge stably while preventing arc discharge and to generate plasma stably in the plasma generation region PR1. This allows realization of effective generation of nitrogen radical NR1 and effective emission of short-wavelength ultraviolet ray 196.

It is further recommended that waveform of the electric pulse should be determined to meet with each of the conditions that increasing rate of voltage V with time (dV/dt) at rising is 50 to 500 kV/$\mu$s, pulse width $\Delta$t is 0.05 to 2.00 $\mu$s, ratio ($V_p$/L1) of peak voltage $V_p$ to distance L1 of the electrode pair is 0.5 to 2.0 kV/cm (center value is 1.0 kV/cm).

For above-mentioned pulse power supply 14 generating the electric pulse, an induction energy store type circuit (hereinafter referred also to as "IES circuit") using static induction type thyristor (hereinafter referred also to as "SIThy") is desirably employed. This IES circuit, in addition to a closing switch function of SIThy, executes turnoff using opening switching function and generates a high voltage across gate and anode of SIThy by the turnoff. Meanwhile, details of the IES circuit are described in "Induction energy store type pulse power supply" by Iida K, Sakuma K presented at 15th SI device symposium (2002).

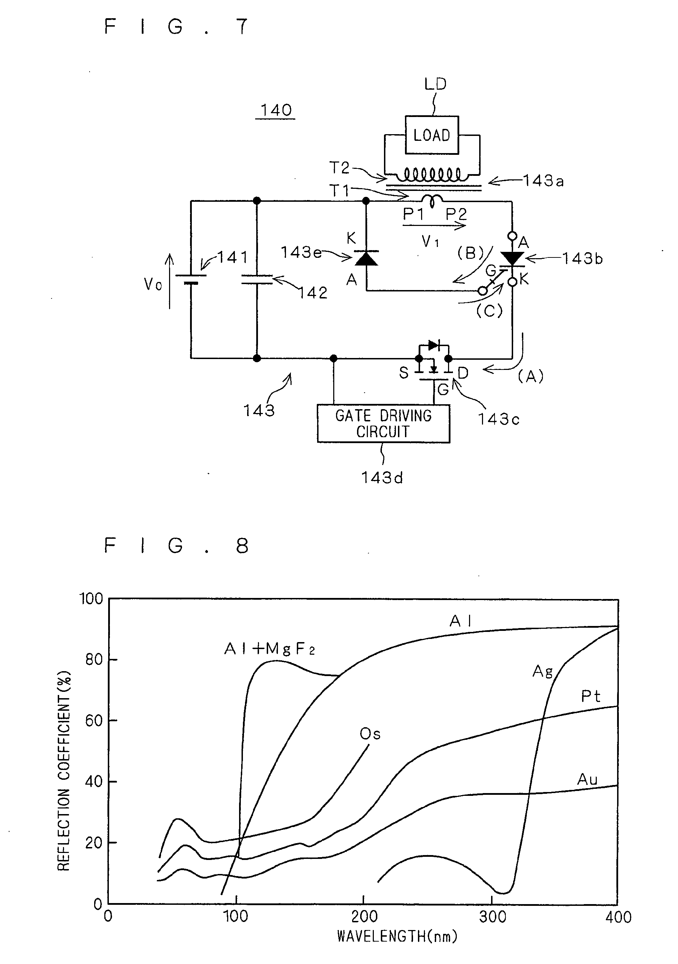

Referring to the circuit diagram shown in FIG. 7, the IES circuit 140 will be explained. The IES circuit 140 includes a low-voltage dc power supply 141 which serves as the current supplying source. Voltage $V_0$ of the low-voltage dc power supply 141 is allowed to be a voltage remarkably lower than peak voltage $V_p$ of the electric pulse generated by the IES circuit 140. For example, even if peak voltage $V_p$ of primary side voltage $V_1$ to be generated at primary side T1 of step up transformer reaches 4 kV, voltage Vo is allowed to be several tens V to hundreds V, typically 40 V to 150 V. The lower limit of this voltage value is determined to be not less than latching voltage of SIThy143b. Since the IES circuit 140 is able to utilize such low-voltage dc power supply as an electric energy source, construction of small-sized and low cost circuit is possible.

To the low-voltage dc power supply 141 are connected a charging capacitor 142 and a step up pulse generation unit 143 in parallel. The charging capacitor 142 intensifies discharging capability of the low-voltage dc power supply 141 by reducing apparent impedance of the low-voltage dc power supply 141. Voltage V0 of the low-voltage dc power supply 141 is stepped up by the step up pulse generation unit 143, while the step up pulse generation unit 143 includes a step up transformer 143a, a SIThy 143b, a MOSFET (Metal Oxide Semiconductor Field Effect Transistor) (hereinafter referred also to as "FET") 143c, a gate driving circuit 143d, and a diode 143e.

In the IES circuit 140, primary side T1 of the step up transformer 143a, anode A - cathode K of SIThy 143b, and drain D - source S of FET 143c are connected in series. That is, one end P1 of primary side T1 of the step up transformer 143a is connected to positive electrode of the low-voltage dc power supply 141, other end P2 of primary side T1 of the step up transformer 143a is connected to anode A of the SIThy 143b, cathode K of the SIThy 143b is connected to drain D of the FET 143c, and source S of the FET 143c is connected to negative electrode of the low-voltage dc power supply 141. This allows that electric current is supplied from the low-voltage dc power supply 141 to these circuit elements. Further, in the IES circuit 140, anode A - gate G of the SIThy 143b is connected in parallel to primary side T1 of the step up transformer 143a via the diode 143e. That is, gate G of the SIThy 143b is connected to anode A of the diode 143e, and cathode K of the diode 143e is connected to one end P1 (positive electrode of low-voltage dc power supply 141) of the diode 143e. A gate driving circuit 143d is connected to gate G - source S of the FET 143c.

The step up transformer 143a is added when the electric pulse given to the primary side T1 is stepped up and output to the secondary side T2. To the secondary side T2 of the step up transformer 143a is connected a load LD (electrode pair 13 in this example). Primary side T1 of the step up transformer 143a is composed of inductive elements having self-inductance.

The SIThy 143b is able to turn ON and turn OFF in response to a signal given to gate G.

The FET 143c is a switching element in which conduction state of drain D - source S changes in response to a signal given from the gate driving circuit 143d. ON voltage or ON resistance of the FET 143c is preferably low. Further, withstand voltage of the FET 143c needs to be higher than the voltage Vo.

The diode 143e is provided to prevent electric current flowing when positive bias is given to gate G of the SIThy 143b, i.e., SIThy 143b is prevented from becoming current driving when positive bias is given to gate G of the SIThy 143b.

Mirror;

The mirror 15 which is a reflection member provided at inner surface of the sealed container 11 has reflection coefficient of short-wavelength ultraviolet ray UV1 not less than 30% and contributes to trapping the short-wavelength ultraviolet ray UV1 emitted by nitrogen atmosphere into aseptization space SP1 and irradiating short-wavelength ultraviolet ray UV1 effectively to the aseptization object substance ST1. Such mirror 15 can be obtained by forming an aluminum film 152 on the glass substrate 151 and by covering the aluminum film with an oxidized silicon ($SiO_x$) film 153 or a magnesium fluoride ($MgF_2$) film 153 in order to improve the durability. When the glass substrate 151 having thickness of 1.0 to 10.0 mm is used in the mirror 15, film thickness of the aluminum film 152 is preferably 100 to 1000 angstrom and film thickness of oxidized silicon film 153 or magnesium fluoride film 153 is preferably 100 to 10000 angstrom. However, since the oxidized silicon film 153 absorbs much short-wavelength ultraviolet ray UV1, film thickness thereof is made thinner as much as possible.

The reason why the aluminum film 152 is selected for the mirror 15 is that, as shown in FIG. 8, reflection coefficient of short-wavelength ultraviolet ray UV1 of the aluminum film 152 is extremely high (about 90%). Meanwhile, FIG. 8 is a graph showing wavelength dependence of reflection coefficient of various metallic films.

Alternatively, in lieu of providing the mirror 15 to inner surface of the sealed container 11, such a configuration may be used that material of the sealed container 11 is changed to metal such as aluminium or ceramics such as alumina, inner surface of the sealed container 11 is mirror finished so that the sealed container 11 also functions as the reflection member.

Far-infrared ray heater

The far-infrared ray heater 16 is mounted to outer surface of the sealed container 11 and raises the temperature of nitrogen atmosphere till 30 to 80˚C, particularly preferably till 70 to 80˚C, by far-infrared ray. With the far-infrared ray heater 16 as mentioned, the aseptization apparatus 1 is now able to hold the temperature of nitrogen atmosphere at a level suitable for aseptization, thereby effectively aseptizing the aseptization object substance ST1. Alternatively, a halogen lamp heater may be used in lieu of the far-infrared ray heater 16.

Carrier

A cage-type carrier 17 for holding and carrying the aseptization object substance ST1 is placed on the cathode 132 and is configured to be capable of moving horizontally in $\pm$ X-direction. This carrier 17 is also designed to be mesh shape or comb teeth shape at least partially so that short-wavelength ultraviolet ray UV1 is prevented from being blocked.
For materials of the carrier 17, for the sake of improvement of durability, use of iron alloy such as stainless steel and tungsten alloy is desirable.

<2.1.2 Operation of aseptization apparatus>

In the aseptization apparatus 1, the aseptization object substance ST1 loaded to the carrier 17 is introduced to the plasma generation region PR1 and an electric pulse is then applied to the electrode pair 13. Then, in the aseptization object substance ST1, cell membrane of bacteria adhered on the surface is exposed to pulse electric field and destroyed, and at the same time, being exposed to nitrogen radical NR1 contained in the plasma generated mostly in the vicinity of the anode 131, and DNA and toxin in the bacteria are destroyed. In addition, short-wavelength ultraviolet ray UV1 having high bactericidal effects is irradiated to the aseptization object substance ST1.
Thanks to composite actions as mentioned, effective aseptization can be carried out in the aseptization apparatus 1 with a small amount of energy.

<2.1.3 Variants>

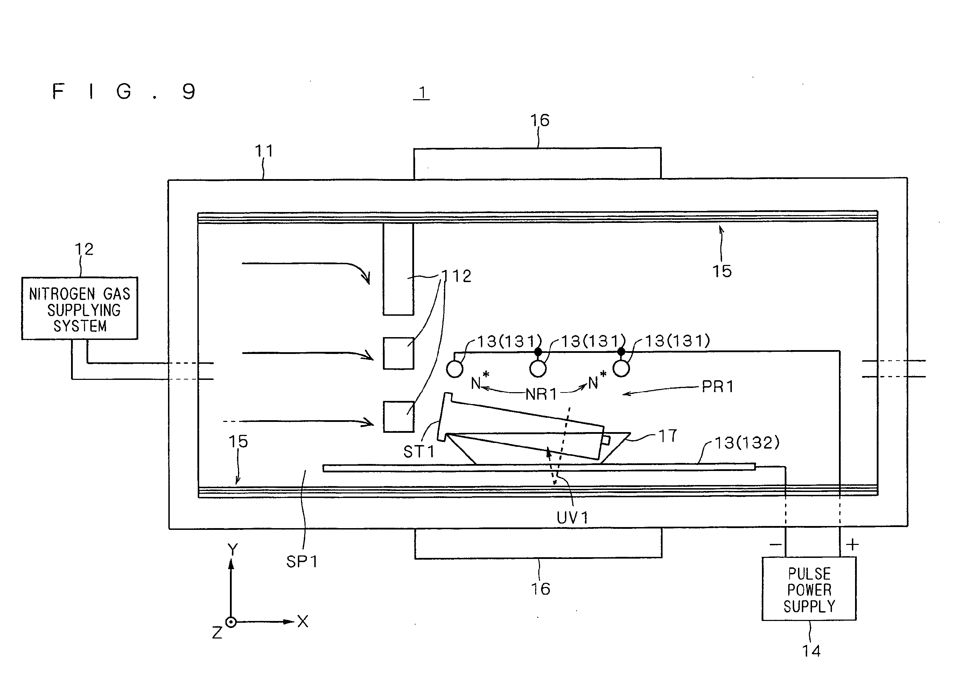

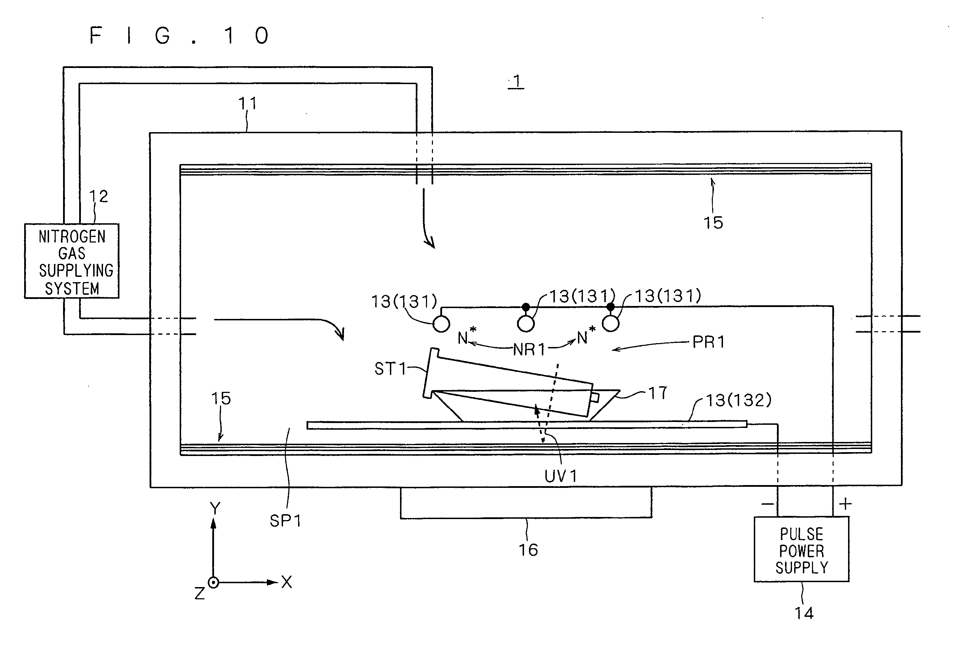

Although, in the first embodiment, the protrusion 111 for disturbing nitrogen gas flow and generating turbulence in the nitrogen atmosphere is formed by immersing upper face of the sealed container 11 towards inside of the aseptization space SP1, as shown in FIG. 9, obstacles 112 for disturbing nitrogen gas flow and generating turbulence in the nitrogen atmosphere may be provided in the aseptization space SP1.
Alternatively, as shown in FIG. 10, such a configuration may be used that nitrogen gas is supplied from two or more locations into the aseptization space SP1 to generate turbulence in the nitrogen atmosphere.

<2.2 Second embodiment>

<2.2.1 Configuration of aseptization apparatus>

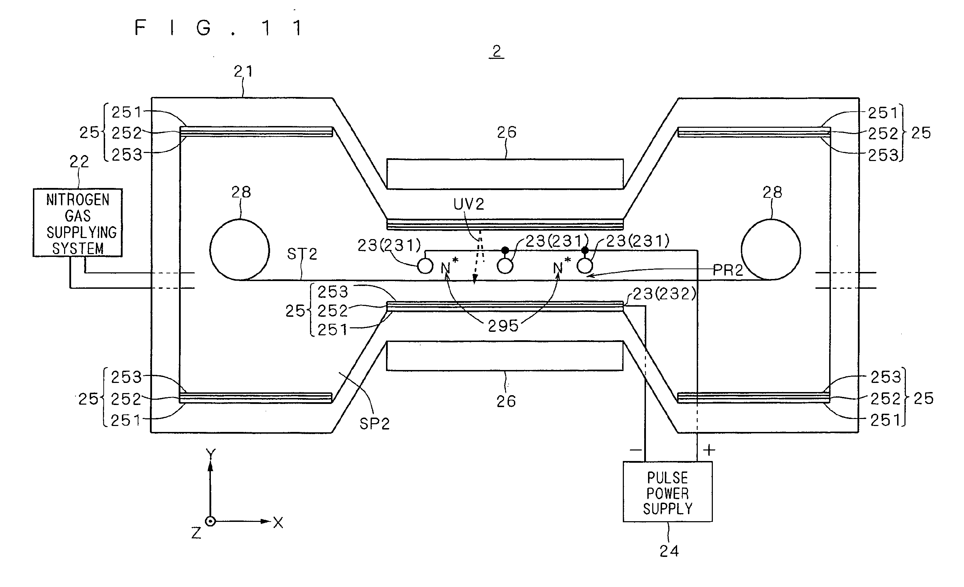

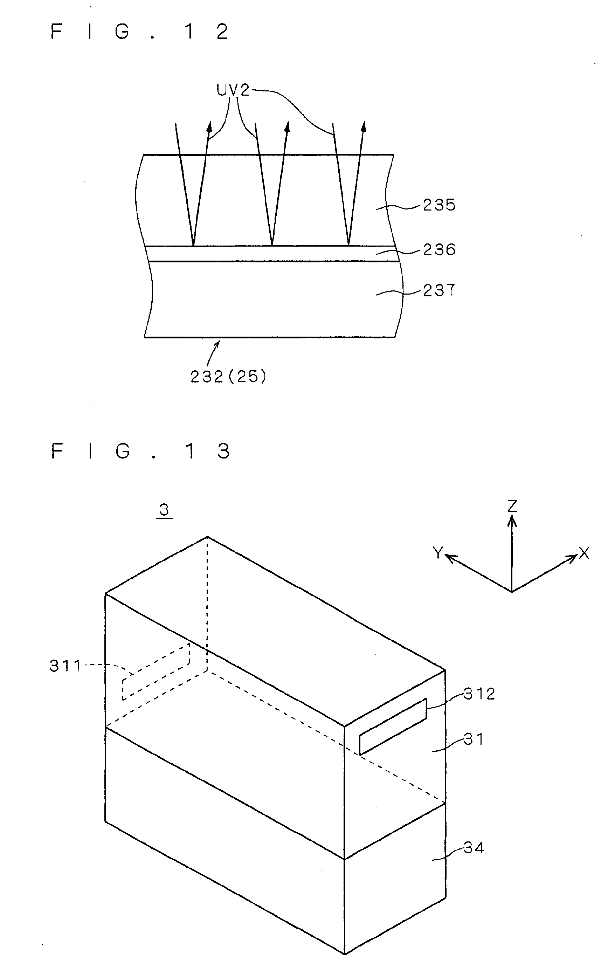

FIG. 11 is a cross-sectional view showing the configuration of the aseptization apparatus 2 according to the second embodiment of the present invention. The aseptization apparatus 2 is configured in such that the surface of an aseptization object substance ST2 having planar shape such as film is aseptized. In FIG. 11, for convenience of explanation, X-Y-Z orthogonal coordinate system is defined in which right and left directions are X-axis direction, up and down directions are Y-axis direction, and fore and aft directions are Z-axis direction.
As shown in FIG. 11, the aseptization apparatus 2 includes a hollow sealed container 21 for forming an aseptization space SP2, a nitrogen gas supplying system 22 which converts atmosphere of the aseptization space SP2 to nitrogen atmosphere, an electrode pair 23 disposed in the aseptization space SP2, a pulse power supply 24 for repeatedly applying an electric pulse to the electrode pair 23, a mirror 25 for returning short-wavelength ultraviolet ray UV2 going from inside of the aseptization space SP2 to outside to inside of the aseptization space SP2, a far-infrared ray heater 26 for controlling a temperature of nitrogen atmosphere, and a roller pair 28 for carrying the aseptization object substance (long film) ST2 inside the aseptization space SP2.
A difference between the aseptization apparatus 2 of the second embodiment and the aseptization apparatus 1 of the first embodiment is such that a carrier for holding and carrying the aseptization object substance is not prepared in the aseptization apparatus 2, while the roller pair 28 for causing the aseptization object substance ST2 to travel horizontally in + X-direction and to pass a plasma generation region PR2 is provided.
The sealed container 21 of the aseptization apparatus 2 has such a configuration that upper face and lower face are immersed towards inside of the aseptization space SP2 in the vicinity of the plasma generation region PR2 being sandwiched by the electrode pair 23. An anode 231 similar to the anode 131 of the first embodiment is provided above horizontal traveling portion of the aseptization object substance ST2, while an aluminum film 252 of the mirror 25 below horizontal traveling portion of the aseptization object substance ST2 is also used as the cathode 232.
Meanwhile, an electrode having cross-sectional structure as shown in the cross-sectional view in FIG. 12 may be used as the cathode 232 which also serves as the mirror 25. That is, as the cathode 232 which also serves as the mirror 25, such an electrode that an aluminum film 236 reflecting the short-wavelength ultraviolet ray UV2 is formed on a transparent substrate 235 such as quartz or glass, and an electric conductor 237 such as aluminum or copper is pasted onto the aluminum film 236, may be used. This cathode 232 is able to effectively reflect the short-wavelength ultraviolet ray UV2 being incident from the direction of the substrate 235 and has excellent characteristics as the electrode.

In the above explanation, explanations of points similar to those of the aseptization apparatus 1 are omitted.

<2.2.2 Operations of aseptization apparatus>

In the aseptization apparatus 2, an electric pulse is applied to the electrode pair 23 opposing each other across the aseptization object substance ST2 while the aseptization object substance ST2 is caused to travel horizontally in the plasma generation region PR2. Then, in the aseptization object substance ST2, cell membrane of bacteria adhered onto the surface is exposed to the pulse electric field and destroyed, and exposed to nitrogen radical NR2 contained in the plasma generated mostly in the vicinity of the anode 231, DNA and toxin in the bacteria are destroyed. In addition, the short-wavelength ultraviolet ray UV2 having high bactericidal effects is irradiated to the aseptization object substance ST2.

Thanks to composite actions as mentioned, effective aseptization can be carried out also in the aseptization apparatus 2 with a small amount of energy.

<2.3 Third embodiment>

<2.3.1 Configuration of aseptization apparatus>

<2.3.1.1 Outline of whole configuration >

FIG. 13 is a perspective view showing whole configuration of an aseptization apparatus 3 according to a third embodiment of the present invention. In FIG. 13, for convenience of explanation, X-Y-Z orthogonal coordinate system is defined in which fore and aft directions (depth direction) are X-axis direction, right and left directions (width direction) are Y-axis direction, and up and down directions (height direction) are Z-axis direction.

The aseptization apparatus 3 shown in FIG. 13 is designed to kill bacteria adhered on a sheet ST3 used as the packing materials for medical devices and food articles, and to destroy DNA and toxin held by the bacteria. Although materials of the sheet ST3 which can be an aseptization object substance of the aseptization apparatus 3 are not specifically limited, for example, plastic resin such as polyethylene, Teflon (registered trademark), polyvinyl chloride, and polyethylene terephthalate is mentioned.

The aseptization apparatus 3 has a housing of nearly rectangular solid shape with dimensions of 600 mm (depth) $\times$ 1400 mm (width) $\times$ 1200 mm (height), and upper part thereof is used as a reactor 31 with 700 mm height and lower part thereof is used as a pulse power supply 34 with 500 mm height.

The aseptization apparatus 3 provides aseptization treatment to a sheet with 300 mm width being thrown to an input port 311 of the reactor 31, and discharges the aseptized sheet ST3 from a discharge port 312 of the reactor 31. A packaging machine for packaging using aseptized sheets is mounted to the aseptization apparatus 3.

<2.3.1.2 Configuration of reactor>

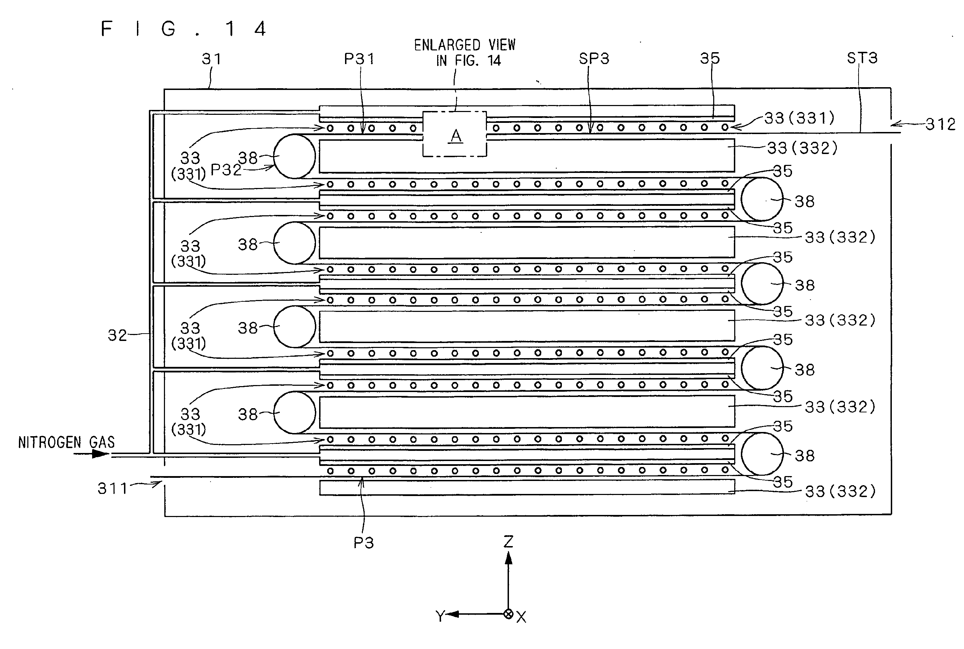

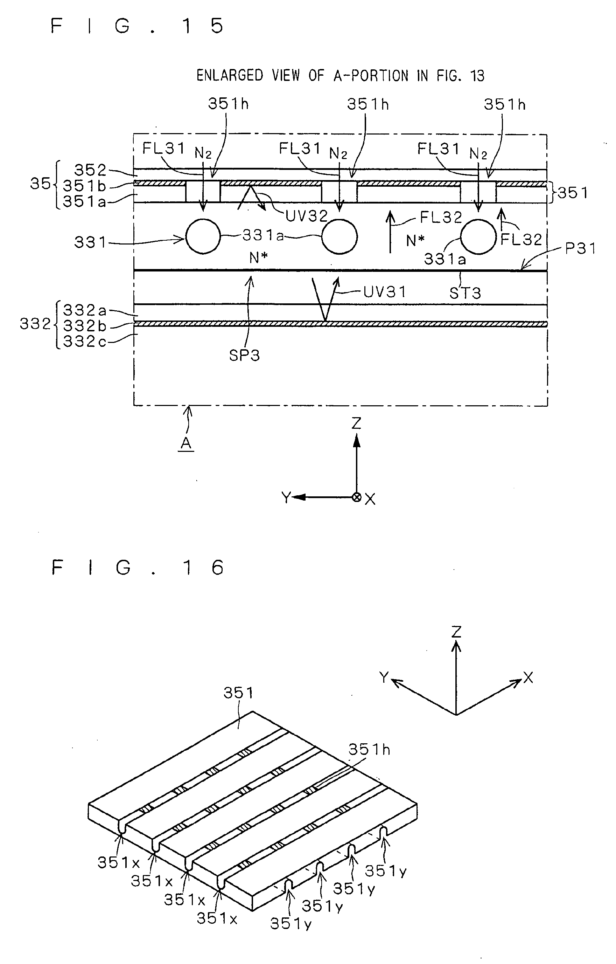

FIG. 14 and FIG. 15 are cross-sectional views showing the configuration of the reactor 31, while FIG. 14 is overall view showing whole of the reactor 31 and FIG. 15 is an enlarged illustration of A-portion shown in FIG. 14.

As shown in FIG. 14, a roller 38 with diameter of 80 mm, to which the sheet ST3 of aseptization object substance is wound by tension, is provided inside the reactor 31. The sheet ST3 is carried from the input port 311 to the discharge port 312 along with a traveling route P3 in zigzag shape including horizontal portion P31 traveling away from the roller 38 and an arc portion P32 traveling very close to the roller 38. In the reactor 31, overall length of the traveling route P3 from the input port 311 to the discharge port 312 is 9 m and traveling velocity of the sheet ST3 is 3 m/min, and therefore, the sheet ST3 input to the input port 311 would be discharged from the discharge port 312 after 3 minutes.

In the reactor 31, the sheet ST3 is aseptized by exposing the sheet ST3 traveling the horizontal portion P31 to the pulse electric field, nitrogen radical and short-wavelength ultraviolet ray, and causing the pulse electric field, nitrogen radical and short-wavelength ultraviolet ray to act on the bacteria adhered on the sheet ST3 compositely.

Electrode pair

The electrode pair 33, which is opposed each other across the sheet ST3 traveling the horizontal portion P31 and is connected to the pulse power supply 34 for generating electric pulse, is provided inside the reactor 31.

Of the electrode pair 33, an anode 331 is configured such that, as shown in FIG. 15, electrode bars 331a with diameter of 0.5 mm are disposed along with the sheet ST3 with 15 mm interval. The electrode bars 331a are disposed so that longitudinal direction thereof becomes ± X-direction that is width direction of the sheet ST3.

Meanwhile, although an idea to replace the electrode plates provided along with the sheet ST3 by the anodes 331 is not discouraged, in this case, in order to prevent such a problem that the anodes 331 block the short-wavelength ultraviolet ray and irradiation of the short-wavelength ultraviolet ray to the sheet 3 is disturbed, electrode plates of mesh shape or comb teeth shape are preferably employed so that opposite side may be seen through.

In the reactor 31, for materials of the anode 331, INCONEL (registered trademark) having excellent durability is employed. However, this does not necessarily discourage that as materials of the anode 331, those other than INCONEL (registered trademark), for example, those composed primarily of metals such as tungsten, molybdenum, manganese, titanium, chrome, zirconium, nickel, silver, iron, copper, platinum and palladium are used. Of course, it is not discouraged that as the anode 331, an electrode bar onto which a film, nitride film, or CVD film composed primarily of these metals is formed using film forming means such as plating is used. Meanwhile, "metal" as used herein includes an alloy containing two or more kinds of metals such as nickel alloy or iron alloy as typically represented by stainless steel.

Of the electrode pair 33, the cathodes 332 are disposed along with the sheet ST3 as shown in FIG. 14.

The cathode 332 is configured such that an aluminum film 332b with thickness of 0.5 to 10.0 $\mu$m is formed by vapor deposition on the lower face of a quartz glass plate 332a with thickness of 0.5 to 5.0 mm. In the cathode 332, upper face of the quartz glass plate 332a onto which the aluminum film 332b is not formed is faced towards the sheet ST3.

The cathode 332 also serves as a mirror for reflecting short-wavelength ultraviolet ray and functions such that a short-wavelength ultraviolet ray UV31 escaping from inside of the space SP3 between the electrode pair 33 to lower part is reflected and is returned to inside of the space SP3. In the aseptization apparatus 3, utilization efficiency of the short-wavelength ultraviolet ray emitted by the nitrogen atmosphere is improved due to providing the cathode 332, and improvement of aseptization efficiency is attempted by increasing the short-wavelength ultraviolet ray acting on the bacteria adhered onto the sheet ST3. Here, formation of a mirror surface by the aluminum film 332b is due to the fact that, as explained in the first embodiment, reflection coefficient of short-wavelength ultraviolet ray of the aluminum film is extremely high (about 90%).

In the cathode 332, in order to ensure electrical continuity, the aluminum film 332b is closely contacted to a hollow aluminum block 332c connected to the pulse power supply 34.

Reflecting plate

Inside the reactor 31, a reflecting plate 35 is provided above the anode 331, which reflects a short-wavelength ultraviolet ray UV32 escaping from inside the space SP3 to upward and returns it to inside the space SP3. The reflecting plate 35 is provided parallel to the sheet ST3.

As shown in FIG. 14, the reflecting plate 35 is configured in such that a laminated mesh 352 made of stainless steel is placed and fixed on the hole drilling mirror 351 being formed by vapor deposition of an aluminum film 351b on upper face of a glass plate 351a. In the reflecting plate 35, lower face of glass plate 351a on which the aluminum film 351b is not formed is directed towards the sheet ST3. In the reactor 31, utilization efficiency of the short-wavelength ultraviolet ray emitted by the nitrogen atmosphere is improved by providing the reflecting plate 35, and improvement of aseptization efficiency is attempted by increasing the short-wavelength ultraviolet ray acting on the bacteria adhered onto the sheet ST3.

Here, formation of a mirror finished surface by the aluminum film 351b is due to the fact that, similar to the case of the cathode 332, reflection coefficient of short-wavelength ultraviolet ray of the aluminum film is extremely high (about 90%).

To the hole drilling mirror 351 are formed penetration holes 351h penetrating the upper face and the lower face regularly in both longitudinal and lateral directions. In the reactor 31, nitrogen gas supplied via a piping 32 is injected towards the sheet ST3 through an opening of the mesh 352 and a penetration hole 351h of the hole drilling mirror 351 to convert atmosphere of the space SP3 where the sheet ST3 is present to nitrogen atmosphere. Thus, generation of a nitrogen gas flow FL31 vertical to the sheet SP3 can prevent reduction in aseptization efficiency and stability due to nitrogen gas accumulation, and at the same time, since chemical species caused by reaction with nitrogen radical N* (hereinafter referred to as "reaction completed chemical species") is eliminated from vicinity of the sheet ST3 by nitrogen gas flow (nitrogen gas flow leaving the sheet ST3) FL32 of blow over of nitrogen gas flow FL 31 going towards the sheet ST3, aseptization can be performed stably at higher efficiency. Further, generation of the nitrogen gas flow FL31 parallel to the pulse electric field has such advantages that uniformity of plasma generation and aseptization can be improved, and generation of ozone which disturbs aseptization can be suppressed to a level which does not pose a practical problem even if the reactor 31 is not sealed completely. This is because if the nitrogen gas flow FL31, which is parallel to the pulse electric field and is going from the anode 331 to the cathode 332, is generated, oxygen gas is hardly mixed thereto. Meanwhile, since emission of pink color light is observed when ozone is generated, presence or absence of ozone generation can be confirmed easily.

As shown in the perspective view in FIG. 16, a plurality of grooves 351x extending in $\pm$ X-direction are excavated on the upper face of the hole drilling mirror 351 at 15 mm interval, and a plurality of grooves 351y extending in $\pm$ Y-direction are excavated on the lower face of the hole drilling mirror 351 at 15 mm interval. On the hole drilling mirror 351, an intersection of the groove 351x and groove 351y orthogonal each other serves as the penetration hole 351h. The hole drilling mirror 351 can be produced in shorter time at lower costs than individual drilling of the penetration hole 351h. Meanwhile, use of the mesh 352 as a pressure drop member of nitrogen gas is not essential, and madreporite of ceramics such as alumina and SiC may be used.

The mesh 352 has mesh spacing of about 0.1 mm and functions as a buffer for uniformizing injection of nitrogen gas from a number of penetration holes 351h. Buffer function thus provided can prevent that nitrogen gas injection is concentrated to the penetration hole 351h at upperstream of the piping 32 thereby disturbing uniformity of aseptization.

The amount of injection of nitrogen gas per 1 $cm^2$ is desirably not less than 0.001 liter/min and no more than 0.03 liter/min. This is because if the amount of injection of nitrogen gas per 1 $cm^2$ is less than 0.001 liter/min, elimination of reaction completed chemical species would become insufficient thereby reducing stability and efficiency of aseptization, and if the amount of injection of nitrogen gas per 1 $cm^2$ is more than 0.03 liter/min, plasma would escape from the space SP3 thereby reducing the amount of emission of short-wavelength ultraviolet ray and aseptization efficiency.

With the reactor 31 having such configuration, electric field of anode side of the sheet ST3 is stronger that that of cathode side, and therefore, aseptization effects on the sheet ST3 is somewhat remarkable at anode side facing to the anode 331 than cathode side facing to the cathode 332.

<2.3.1.3. Configuration of pulse power supply>

Waveform of the electric pulse applied repeatedly to the electrode pair 33 by the pulse power supply 34 is determined so that destruction of cell membrane of the bacteria is possible by the pulse electric field, fine streamer discharge is induced without inducing arc discharge, and plasma can be generated stably in the space SP3. Concrete waveform of the electric pulse will be explained in "2.3.2 Operations of aseptization apparatus" described below.

The pulse power source 34 used as mentioned above preferably employs the IES circuit 140 explained in the first embodiment.

<2.3.2 Operations of aseptization apparatus>

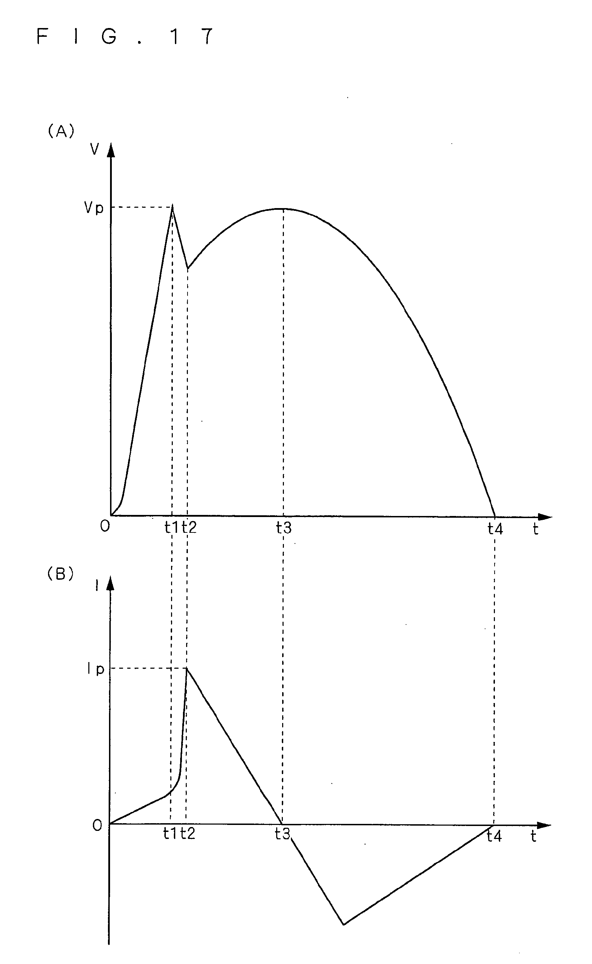

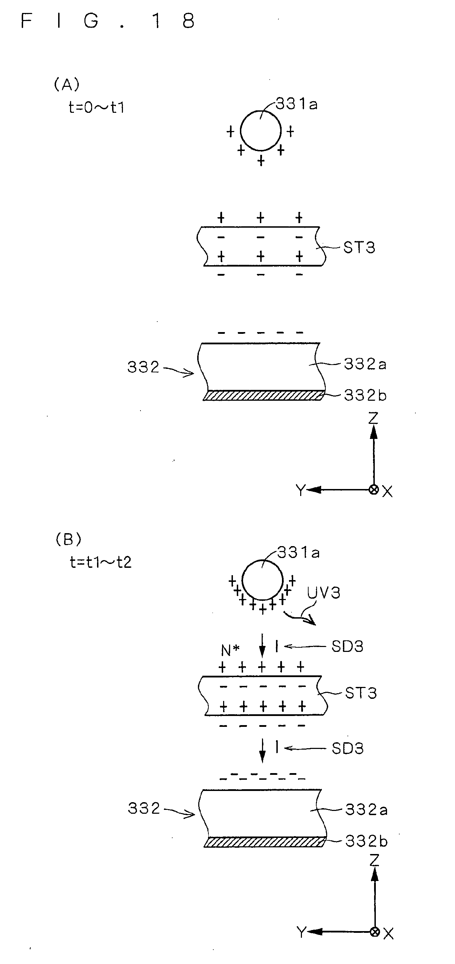

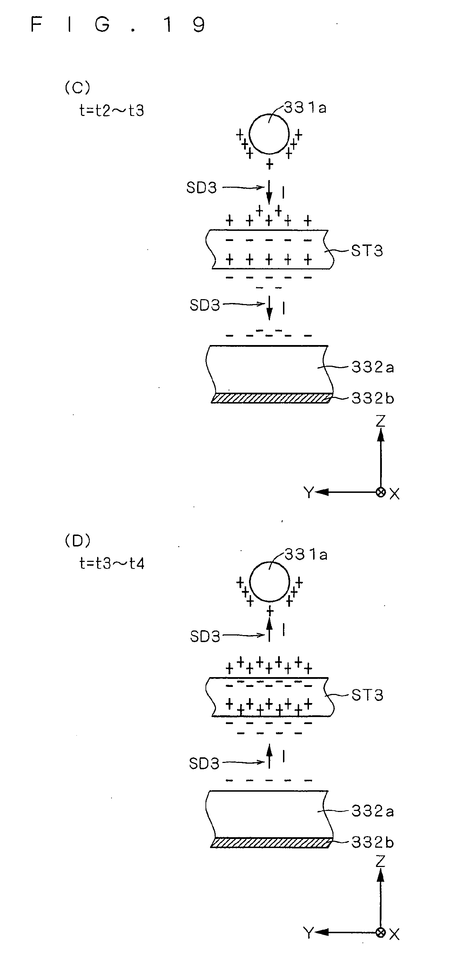

The following description explains operations of the aseptization apparatus 3 referring to FIG. 17 to FIG. 19. FIG. 17 is a drawing showing rough waveform of electric pulse to be applied repeatedly to the electrode pair 33, FIG. 17 (A) and FIG. 17 (B) show changes in voltage V across the electrode pair 33 and in electric current I flowing into the anode 331 with lapse of time t, respectively. Further, FIG. 18 and FIG. 19 are drawings showing a state of the space SP3 when the electric pulse is applied to the electrode pair 33, and FIG. 18 (A), FIG. 18 (B), FIG. 19 (C) and FIG. 19 (D) show a state of the space SP3 at voltage rising step (t = 0 to t1), discharging step (t = t1 to t2), discharging termination step (t = t2 to t3), and reverse discharge step (t = t3 to t4), respectively.

In the aseptization apparatus 3, since, in the cathode 332, the aluminum film 332b is covered with the quartz glass plate 331a that functions as a dielectric body barrier, when the electric pulse is applied, a time before the electric current I is discontinued becomes longer. For this reason, input electric power I x V increases in the reactor 10, temperature of nitrogen atmosphere increases up to temperature suitable for aseptization, for example, about 48˚C for stearothermophilus case, and aseptization can be performed effectively.

Voltage rising step (t = 0 to t1);

As shown in FIG. 17, in the voltage rising step, voltage V rises rapidly in a short time of 50 to 100 ns and reaches peak voltage Vp, while no fine streamer discharge occurs in the space SP3 (FIG. 18 (A)), and electric current I simply increases gently resulting from charging to electrostatic capacitance across the electrode pair 33.

Meanwhile, increasing rate of voltage V with time (dV/dt) at rising is desirably in a range of 100 to 500 kV/$\mu$s.

Further, peak voltage $V_p$ is, depending on a distance between the anode 331 and the cathode 332, typically in a range of 10 to 30 kV, and is about 15 kV for a case where the distance between the anode 331 and the cathode 332 is 4.5 mm.

By applying an electric pulse with quick rising to the electrode pair 33, in the aseptization apparatus 3, the sheet ST3 traveling the horizontal portion P31 is exposed to the pulse electric field, and the pulse electric field is caused to act on bacteria adhered onto the sheet ST3 to destroy cells of the bacteria.

Discharging step (t = t1 to t2);

As shown in FIG. 17, in the discharging step subsequent to the voltage rising step, voltage V decreases more or less resulting from fine streamer discharge SD3 occurred in the space SP3 (FIG. 18 (B)), while electric current Ip increases rapidly to reach peak electric current I (10 to 50A).

In the discharging step, an electronic avalanche moving from the cathode 332 to the anode 331 resulting from fine streamer discharge SD3 is accelerated, and plasma is generated in the nitrogen atmosphere around the anode 331. In the discharging step, the sheet ST3 is exposed to nitrogen radical N* contained in the plasma thus generated, nitrogen radical is then caused to act on bacteria adhered onto the sheet ST3 to destroy DNA of the bacteria.

Further, in the discharging step, short-wavelength ultraviolet ray emitted by the nitrogen atmosphere resulting from fine streamer discharge SD3 is irradiated to the sheet ST3, and the short-wavelength ultraviolet ray UV3 can be caused to act on bacteria adhered to the sheet ST3. [0155]Discharging termination step (t = t2 to t3);

As shown in FIG. 19 (C), in the discharging termination step subsequent to the discharging step, fine streamer discharge SD3 terminates with lapse of time. Therefore, in the discharging termination step, voltage V increases slightly and electric current I decreases gently (FIG. 17). [0156]Reverse discharging step (t = t3 to t4)

As shown in FIG. 19 (D), in the reverse discharging step subsequent to the discharging termination step, fine streamer discharge SD3 in the reverse direction of the discharging step occurs. This results in reduction in voltage V and electric current I flows in the reverse direction of the discharging step (FIG. 17). In other words, in the reverse discharging step, roles of the anode 331 and the cathode 332 are substantially reversed.

Thus, when electric current flowing in response to application of the electric pulse to the electrode pair 33 is reversed, in the reactor 31, electrostatic charge of the sheet ST3 is neutralized, thereby preventing charge-up of the sheet ST3. This allows that unevenness of fine streamer discharge SD3 resulting from charge-up of the sheet ST3 can be prevented, and aseptization of the sheet ST3 is uniformed.

<2.3.3 Relationship between flow rate of nitrogen gas and aseptization efficiency>

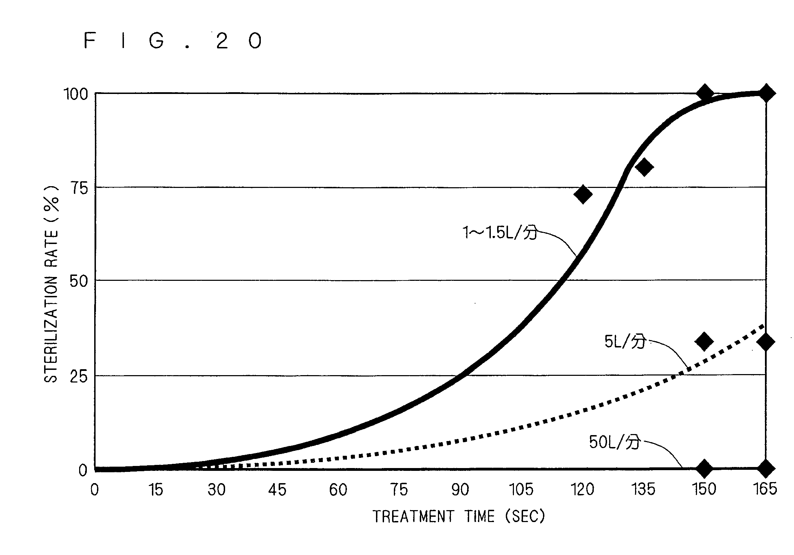

Next, referring to FIG. 20, a relationship between flow rate of nitrogen gas and aseptization efficiency will be explained. FIG. 20 is a drawing showing the relationship between flow rate of nitrogen gas and aseptization efficiency in the reactor with bottom area of 600 mm$^2$ and height of 100 mm. Dependency of aseptization efficiency (vertical axis) on treatment time (horizontal axis) is shown against nitrogen gas flow rate (1.0 to 1.5 L/min, 5 L/min, 50 L/min) in graphs.

As it is apparent from FIG. 20, as nitrogen gas flow rate increases, aseptization efficiency decreases. This is attributable to that when nitrogen gas flow rate is increased, nitrogen radicals generated are run out thereby disturbing light emission of the short-wavelength ultraviolet ray. Experimental data shown in FIG. 20 prove contribution of nitrogen radical and short-wavelength ultraviolet ray to aseptization.

<2.3.4 Description of sheet>

In the aseptization apparatus 3, an electric pulse is applied to the electrode pair 33 opposing each other across the sheet ST3, fine streamer discharge is induced in the space SP3, and pulse electric field, nitrogen radical, and short-wavelength ultraviolet ray are caused to act on bacteria adhered to the sheet ST3 for aseptization of the sheet ST3. Such aseptization can be adopted since glow discharge is not disturbed by the sheet ST3 so far as thickness of the sheet ST3 is not extremely thickened, for example, so far as thickness of the sheet ST3 is not more than several millimeters.

With the aseptization apparatus 3, both faces of the sheet ST3 can be aseptized with a smaller amount of energy than those used in the ultraviolet ray irradiation method and the electron beam irradiation method, by causing pulse electric field, nitrogen radical, and short-wavelength ultraviolet ray to act on bacteria which are present on the electrode surface of the sheet ST3 compositely. Further, such a problem that change of properties is caused in the direction from treated surface of the sheet ST3 towards more than 0.1 $\mu$m depth direction, which was cited as problematic with the gamma-radiation method, ultraviolet ray irradiation method, and the electron beam irradiation method, is not induced. This is attributable to that short-wavelength ultraviolet ray, which is an aseptization means, hardly permeates deep into the resin sheet. In particular, in the aseptization apparatus 3, excessive change of properties of the sheet ST3 does not occur even if the sheet ST3 is made of polypropylene.

<2.3.5 Variants>

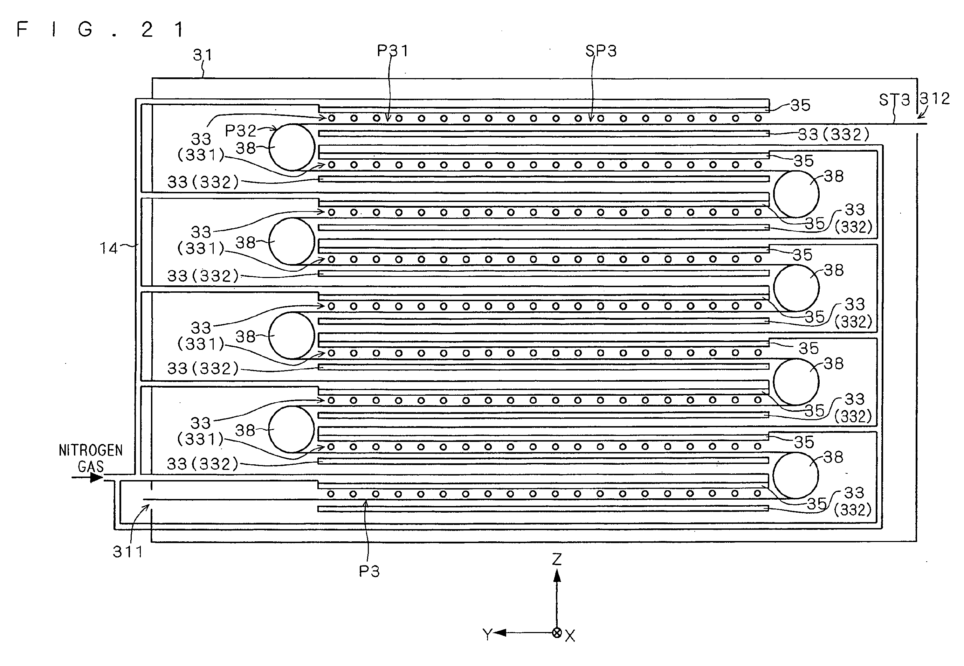

In the third embodiment, aseptization effects on the sheet ST3 becomes slightly remarkable at anode side facing to the anode 331 than cathode side facing to the cathode 332. Therefore, as shown in cross-sectional view in FIG. 21, by exchanging upper/lower relationship of the anode 331 and the cathode 332 in opposed electrode pair 33 at the adjoining stage, and if such a consideration is given so that each of front and rear of the sheet ST3 faces alternately with the anode 331 and the cathode 332, while the sheet ST3 is traveling on the traveling route P3, aseptization effects at the sheet ST3 can be uniformized at the front and the rear.

<2.4 Fourth embodiment>

<2.4.1 Configuration of aseptization apparatus>

<2.4.1.1 Outline of whole configuration>

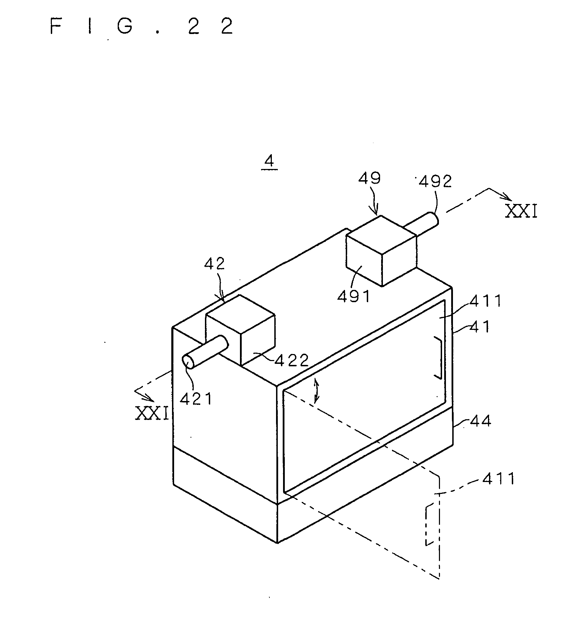

FIG. 22 is a perspective view showing rough configuration of whole aseptization apparatus 4 according to a fourth embodiment of the present invention.

The aseptization apparatus 4 shown in FIG. 22 is a small-sized aseptization apparatus for aseptizing a grinding stone with shaft for dental use.

As shown in FIG. 22, the aseptization apparatus 4 includes a reactor 41 in which aseptization is performed inside, a nitrogen gas introduction unit 42 for introducing nitrogen gas into the reactor 41, a pressure reducing unit 49 for reducing the pressure in the reactor 41, and a pulse power supply 44 for supplying an electric pulse to the reactor 41.

The reactor 41 is designed to be a batch-type reaction container for aseptizing a grinding stone with shaft for dental use accommodated therein. While a sealed door 411 provided at front face is in open state (dotted line), the reactor 41 is in a state where it is possible to accommodate the grinding stone with shaft for dental use inside and take-out the grinding stone with shaft for dental use from inside, while the sealed door 411 is in closed state (solid line), inside thereof is in sealed state.

The nitrogen gas introduction unit 42 introduces nitrogen gas being supplied to an intake port 421 inside the reactor 41 after the temperature is regulated using a heater 422 for temperature regulation.

The pressure reducing unit 49 discharges the nitrogen gas in the reactor 41 using a pressure reducing pump 491 from an exhaust port 492. Inside of the reactor 41 is desirably put into a pressure reduced state in a range of 13 to 1300 Pa (reduced pressure nitrogen atmosphere) by the pressure reducing pump 491. When the pressure inside the reactor 41 is reduced lower than this range, generation of nitrogen radical and short-wavelength ultraviolet ray is suppressed, and aseptization effects are reduced; when the pressure inside the reactor 41 is more than this range, discharge distance becomes shorter and three-dimensional aseptization of the grinding stone with shaft for dental use becomes difficult.

<2.4.1.2 Configuration of reactor>

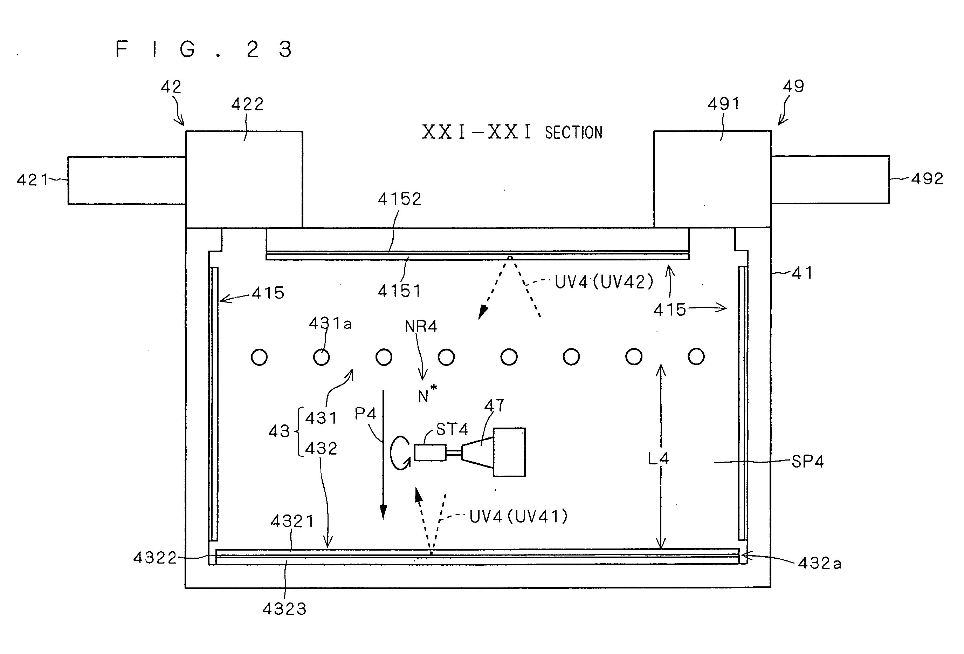

FIG. 23 is a cross-sectional view schematically showing the configuration of the reactor 41. FIG. 23 is a cross-sectional view showing cross-section of the reactor 41 at XXI-XXI section in FIG. 5.

As shown in FIG. 23, an electrode pair 43 connected to the pulse power supply 44 is disposed in a space SP4 inside the reactor 41 where aseptization of a grinding stone with shaft for dental ST4 is performed (hereinafter referred to as "aseptization space"). Between the electrode pair 43, a 3-pawl chuck 47 which grasps the shaft of the grinding stone with shaft for dental and rotates the grinding stone with shaft for dental around the rotating shaft vertical to the pulse electric field P4 is provided. Although one 3-pawl chuck 47 is depicted in FIG. 23, such a configuration may be used that two or more two 3-pawl chucks 47 are provided to allow simultaneous aseptization of two or more grinding stones with shaft for dental use ST4. Of course, a method for grasping the grinding stone with shaft for dental use ST4 should be altered appropriately depending on the profile thereof, and use of 3-pawl chuck 47 is not necessarily required.

With the configuration as mentioned, in the reactor 41 in which aseptization space SP4 is filled with nitrogen atmosphere, when an electric pulse is applied to the electrode pair 43 in a state where the grinding stone with shaft for dental use ST4 is present between the electrode pair 43, (1) pulse electric field P4 generated by electric pulse application to the electrode pair 43, (2) nitrogen radical NR4 contained in the plasma generated in nitrogen atmosphere resulting from fine streamer discharge, and (3) short-wavelength ultraviolet ray UV4 emitted by nitrogen atmosphere resulting from fine streamer discharge do act on bacteria adhered onto the grinding stone with shaft for dental use ST4, and the grinding stone with shaft for dental use ST4 is aseptized.

Further, in the reactor 41, fine streamer discharge is induced in the nitrogen atmosphere pressure reduced and made lean down to 1/10 to 1/2 of the atmospheric pressure (10000 MPa to 50000 MPa), in order to lengthen the discharge distance and secure the spacing L4 of the electrode pair 43 (typically, 25 to 50 mm that is more than five times that of atmospheric pressure case) that enables three-dimensional aseptization of the grinding stone with shaft for dental use ST4. In addition, inducing fine streamer discharge in the pressure reduced and made lean nitrogen atmosphere contributes to lengthening of lifetime of nitrogen radical NR4 (typically more than 10 times that of atmospheric pressure case) and to effective aseptization of the grinding stone with shaft for dental use ST4.

Further, in the reactor 41, chemical species generated by reaction of nitrogen radical NR4 are eliminated from the aseptization space SP4 by an exhaust unit 40 and replaced by fresh nitrogen gas, and therefore, aseptization can be performed at high efficiency.

An anode 431 constituting the electrode pair 43 is configured in such that a plurality of electrode bars 431a having diameter of 0.5 mm are disposed horizontally at a constant interval, and a cathode 432 constituting the electrode pair 43 is configured in such that one sheet of electrode plate 432a is disposed horizontally.

In the reactor 41, for materials of the anode 431, INCONEL (registered trademark) having excellent durability is employed. However, this does not discouraged that for materials of the anode 431, those other than INCONEL (registered trademark), for example, those composed primarily of metals such as tungsten, molybdenum, manganese, titanium, chrome, zirconium, nickel, silver, iron, copper, platinum and palladium are used. Of course, as the anode 431, use of an electrode bar onto which a metallic film composed primarily of these metals or nitride film is formed using metallic film forming means such as plating or CVD (chemical vapor deposition) is not discouraged. Meanwhile, "metal" as used herein includes an alloy containing two or more kinds of metals such as nickel alloy or iron alloy as typically represented by stainless steel.

Although it is not discouraged that the anode 431 is configured of an electrode plate, in this case, in order to prevent that the anode 431 disturbs short-wavelength ultraviolet ray UV4 and irradiation of short-wavelength ultraviolet ray UV4 to the grinding stone with shaft for dental use ST4, the electrode plate is desirably in mesh shape or comb teeth shape so that opposite side may be seen through.

The cathode 432 is configured in such that an aluminum film 4322 with thickness of 0.5 to 10.0 $\mu$m is formed by vapor deposition onto one principal surface of a quartz glass plate 4321 with thickness of 0.5 to 5.0 mm. In the cathode 432, another principal surface, onto which the aluminum film 4322 is not formed, of the quartz glass plate 4321 is directed towards the grinding stone with shaft for dental use ST4.

In the cathode 432, in order to ensure electric continuity, the aluminum film 4322 is closely contacted to a metallic electrode 4323 connected to the pulse power supply 44.

The cathode 432 provided to inner surface at lower part of the reactor 41 also serves as a mirror for reflecting short-wavelength ultraviolet ray UV4 and functions such that a short-wavelength ultraviolet ray UV41 escaping from inside of the aseptization space SP4 to lower part is reflected and is returned to inside of the aseptization space SP4. In the reactor 41, utilization efficiency of the short-wavelength ultraviolet ray UV4 generated in the nitrogen atmosphere is improved by providing such cathode 432 and improvement of aseptization efficiency is attempted by increasing the short-wavelength ultraviolet ray UV4 acting on the bacteria adhered onto the grinding stone with shaft for dental use ST4. Here, formation of a mirror finished surface by the aluminum film 4322 is due to the fact that, as explained in the first embodiment, reflection coefficient of short-wavelength ultraviolet ray UV4 of the aluminum film is extremely high (about 90%).

With the use of such anode 431 and cathode 432, although electric field becomes stronger as coming closer to the anode 431 and aseptization efficiency also becomes somewhat higher as coming closer to the anode 431, in the reactor 41, it is possible to sequentially direct whole grinding stone with shaft for dental use ST4 towards the anode 431 side with higher aseptization efficiency by causing the grinding stone with shaft for dental use ST4 to rotate, and accordingly, whole grinding stone with shaft for dental use ST4 can be aseptized uniformly.

Further, in the inner surface of upper part and side part of the reactor 41 is pasted a reflecting plate 415 which reflects a short-wavelength ultraviolet ray UV42 escaping from inside of the aseptization apparatus SP4 to upper part and side part and returns it to inside of the aseptization apparatus SP4. The reflecting plate 415 is configured in such that an aluminum film 4152 with thickness of 0.5 to 10.0 $\mu$m is film formed by vapor deposition onto one principal surface of a quartz glass plate 4151 with thickness of 0.5 to 5.0 mm, similar to the case of the cathode 432. In reflecting plate 415, also, another principal surface, onto which the aluminum film 4152 is not film formed, of the quartz glass plate 4151 is directed towards the grinding stone with shaft for dental use ST4. In the reactor 41, utilization efficiency of the short-wavelength ultraviolet ray UV4 emitted by the nitrogen atmosphere is improved by providing the reflecting plate 415 and improvement of aseptization efficiency is attempted by increasing the short-wavelength ultraviolet ray UV4 acting on the bacteria adhered onto the grinding stone with shaft for dental use ST4.

Again, formation of a mirror finished surface by the aluminum film 4152 is due to the fact that, similar to the case of the cathode 432, reflection coefficient of the short-wavelength ultraviolet ray UV4 of the aluminum film 4152 is extremely high (about 90%).

In the reactor 41, although temperature rise of nitrogen atmosphere due to discharge contributes to improvement of the aseptization efficiency, use of the heater 422 for temperature regulation of nitrogen atmosphere is desirable to bring nitrogen atmosphere temperature to a temperature most suited for aseptization. When the temperature most suited for aseptization is lower than that of atmospheric pressure case (e.g., stearothermophilus) due to pressure reduction in the aseptization space SP4, temperature of the nitrogen atmosphere should be determined paying attention to that it is reduced from about 58˚C for atmospheric pressure case to 30˚C to 50˚C. Of course, when temperature rise of the nitrogen atmosphere due to discharge is excessive, installation of a cooler in lieu of the heater 422 as means for temperature regulation is desirable to cool the nitrogen gas introduced to the aseptization space SP4.

<2.4.1.3 Configuration of pulse power supply>

Waveform of the electric pulse applied repeatedly by the pulse power source 44 to the electrode 43 is determined so that cell membrane of bacteria can be destroyed by the pulse electric field and at the same time, fine streamer is induced without inducing arc discharge, and plasma can be generated stably in the space SP4. Concrete waveforms of the electric pulse will be explained in "2.4.2 Operations of aseptization apparatus" described below.
The IES circuit 140 as explained in the first embodiment is desirably employed in the pulse power supply 44 as mentioned above.

<2.4.2 Operations of aseptization apparatus>

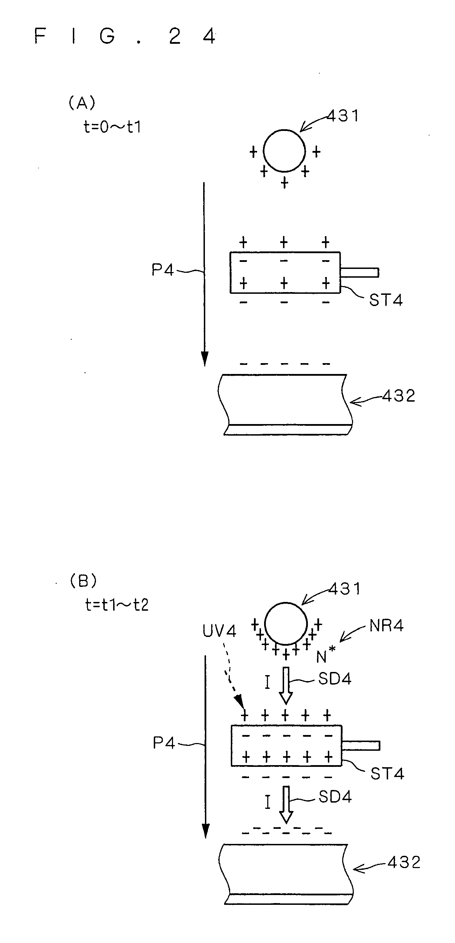

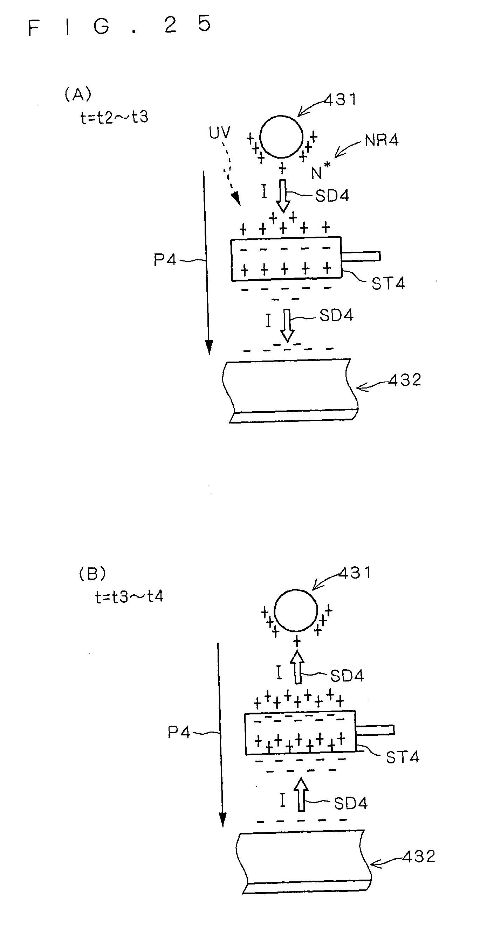

Referring now to FIG. 17, FIG. 24 and FIG. 25, operations of the aseptization apparatus 4 will be explained below. FIG. 17 is a drawing showing rough waveform of electric pulse as much as one pulse to be applied repeatedly to the electrode pair 43, and FIG. 17 (A) and FIG. 17 (B) show changes in voltage V across the electrode pair 43 and in electric current I flown into the anode 432 with lapse of time t, respectively. FIG. 24 and FIG. 25 are drawings showing a state of the space SP4 when an electric pulse is applied to the electrode pair 43, and FIG. 24 (A), FIG. 24 (B), FIG. 25 (A) and FIG. 25 (B) show a state of the space SP4 at voltage rising step (t = 0 to t1), discharging step (t = t1 to t2), discharging termination step (t = t2 to t3), and reverse discharge step (t = t3 to t4), respectively.
In the aseptization apparatus 4, since, in the cathode 432, the aluminum film 4322 is covered with the quartz glass plate 4321 that functions as a dielectric body barrier, when the electric pulse is applied, a time before the electric current I is discontinued becomes longer. For this reason, input electric power $I \times V$ increases in the reactor 41, temperature of nitrogen atmosphere increases and aseptization can be performed effectively. [0188]Voltage rising step (t = 0 to t1);
As shown in FIG. 17, in the voltage rising step, voltage V rises rapidly in a short time of 50 to 100 ns and reaches peak voltage $V_p$, while no fine streamer discharge occurs in the space SP4 (FIG. 24 (A)), and electric current I simply increases gently resulting from charging to electrostatic capacitance across the electrode pair 43.
Meanwhile, increasing rate of voltage V with time (dV/dt) at rising is desirably in a range of 100 to 500 kV/$\mu$s.
Further, peak voltage $V_p$ is, depending on a distance L4 between the electrode pair 43, 5 to 15 kV for a case where the distance L4 is 50 to 100 mm.
By applying an electric pulse with fast rising to the electrode pair 43, in the aseptization apparatus 4, the grinding stone with shaft for dental use ST4 is exposed to the pulse electric field P4 and cell membrane of bacteria is destroyed by causing the pulse electric field P4 to act on the bacteria adhered to the grinding stone with shaft for dental use ST4.

Discharging step (t = t1 to t2);

As shown in FIG. 17, in the discharging step subsequent to the voltage rising step, voltage V decreases more or less resulting from fine streamer discharge SD4 occurred in the space SP4 (FIG. 24 (B)), while electric current I increases rapidly to reach peak electric current $I_p$.
In the discharging step, an electronic avalanche moving from the cathode 331 to the anode 332 resulting from fine streamer discharge SD4 is accelerated, and plasma is generated in the nitrogen atmosphere around the anode 332. In the discharging step, the grinding stone with shaft for dental use ST4 is exposed to nitrogen radical NR4 contained in the plasma thus generated, nitrogen radical NR is then caused to act on bacteria adhered onto the grinding stone with shaft for dental use ST4 to destroy DNA of the bacteria.
Further, in the discharging step, short-wavelength ultraviolet ray UV4 emitted by the nitrogen atmosphere resulting from fine streamer discharge SD4 is irradiated to the grinding stone with shaft for dental use ST4, and the short-wavelength ultraviolet ray can be caused to act on bacteria adhered to the grinding stone with shaft for dental use ST4.

Discharging termination step (t = t2 to t3);

As shown in FIG. 25 (A), in the discharging termination step subsequent to the discharging step, fine streamer discharge SD4 terminates with lapse of time. Therefore, in the discharging termination step, voltage V increases slightly and electric current I decreases gently (FIG. 17). [0196]Reverse discharging step (t = t3 to t4);
As shown in FIG. 25 (B), in the reverse discharging step subsequent to the discharging termination step, fine streamer discharge SD4 in the reverse direction of the discharging step occurs. This results in reduction in voltage V and electric current I flows in the reverse direction of the discharging step (FIG. 17). In other words, in the reverse discharging step, roles of the anode 431 and the cathode 432 are substantially reversed from those of the discharging step.

Thus, by causing reversal of the electric current flowing in response to application of the electric pulse to the electrode pair 43, in the reactor 41, electrostatic charge of the grinding stone with shaft for dental use ST4 is neutralized, thereby preventing charge-up of the grinding stone with shaft for dental use ST4. This allows that unevenness of fine streamer discharge SD4 resulting from charge-up of the grinding stone with shaft for dental use ST4 is prevented, and aseptization of the grinding stone with shaft for dental use ST4 is uniformed. Of course, the 3-pawl chuck 47 is desirably grounded in advance for appropriate prevention of charge-up.

With the aseptization apparatus 4 as mentioned, the grinding stone with shaft for dental use ST4 can be aseptized with a smaller amount of energy than those used in the electron beam irradiation method, heating method, and ultraviolet ray irradiation method, by causing pulse electric field, nitrogen radical, and short-wavelength ultraviolet ray to act on bacteria which are present on the electrode surface of the sheet ST4 compositely, and still the grinding stone with shaft for dental use ST4 is not damaged.

<2.4.3 Variants>

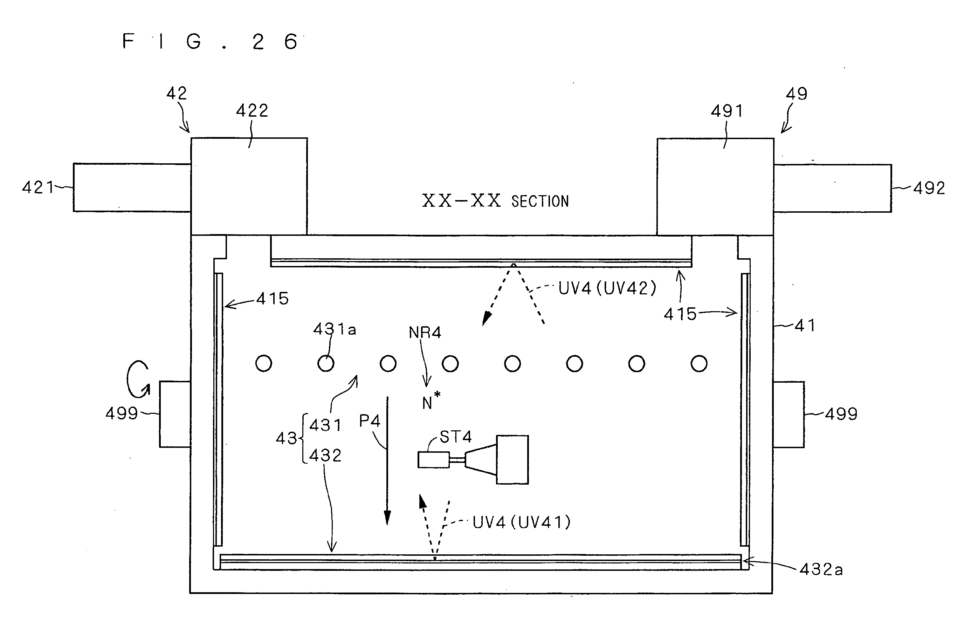

In the fourth embodiment, although an example where the grinding stone with shaft for dental use ST4 is caused to rotate is exemplified, whole grinding stone with shaft for dental use ST4 can be aseptized by such a configuration that, as shown in FIG. 26, whole reactor 41 is turned around the rotating shaft vertical to the pulse electric field P4 by a rotary mechanism 499 and the anode 431 and the cathode 432 revolve around the grinding stone with shaft for dental use ST4 at the same cycle, while the grinding stone with shaft for dental use ST4 is fixed.

Further, although the aseptization apparatus 4 for aseptizing the grinding stone with shaft for dental use ST4 is explained in the fourth embodiment, an aseptization object substance which the aseptization apparatus 4 intends to target is not limited to the grinding stone with shaft for dental use ST4. Generally speaking, the aseptization apparatus 4 is an aseptization apparatus for aseptizing three-dimensional non-planar articles having the same size in each of directions of three dimensions and is capable of aseptizing three-dimensional article such as packaging materials for medical devices and medical instrumentations other than the grinding stone with shaft for dental use ST4.

<2.5 Fifth embodiment>

<2.5.1 Configuration of aseptization apparatus>

<2.5.1.1 Outline of whole configuration>

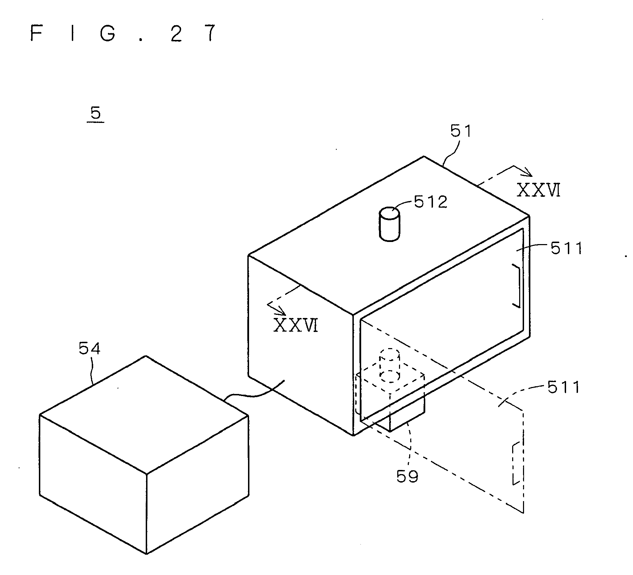

FIG. 27 is a perspective view showing outline of whole configuration of aseptization apparatus 5 according to the fifth embodiment of the present invention.

The aseptization apparatus 5 shown in FIG. 27 is a small-sized aseptization apparatus for aseptizing three-dimensional non-planar articles having the same size in each of directions of three dimensions.

As shown in FIG. 27, the aseptization apparatus 5 includes a reactor 51 in which aseptization is performed, a pump 59 for pressure reducing the inside of the reactor 51, and a pulse power supply 54 for supplying electric pulse to the reactor 51.

The reactor 51 is designed to be a batch-type reaction container for aseptizing an aseptization object substance ST5 accommodated therein. A nitrogen gas intake port 512 is provided on upper face of the reactor container of the reactor 51, and a nitrogen gas exhaust port 513 is provided on the lower face thereof. While a sealed door 511 provided at front face is in open state (alternate long and two short dashed lines), the reactor 51 is in a state where it is possible to accommodate the aseptization object substance ST5 to inside and take-out the aseptization object substance ST5 from inside, while the sealed door 511 is in closed state (solid line), inside thereof is in sealed state.

The reaction container of the reactor 51, particularly the sealed door 511, is desirably made of raw materials such as stainless steel which blocks electromagnetic wave. This is to prevent leakage of electromagnetic wave generated at application of electric pulse to the electrode pair 53 inside the reactor 51 and to prevent bad influences by the electromagnetic wave upon workers and electronic equipment present around the reactor 51. It is desirable to paste metallic gauzes, films, or the like for electromagnetic shielding on exterior or interior of the reaction container of the reactor 51, even if the reaction container of the reactor 51 is configured by raw materials such as polycarbonate which does not shield the electromagnetic wave in order to permit visual confirmation of inside of the reactor 51.

Further, the reaction container of the reactor 51 is preferably composed by raw materials which shield the short-wavelength ultraviolet ray. This is to prevent leakage of short-wavelength ultraviolet ray generated at application of electric pulse to the electrode pair 53 in the reactor 51 and to prevent bad influences by the short-wavelength ultraviolet ray upon workers and electronic equipment present around the reactor 51.

The pump 59 discharges the nitrogen gas inside from the exhaust port 513 of the reactor 51.

<2.5.1.2 Configuration of reactor>

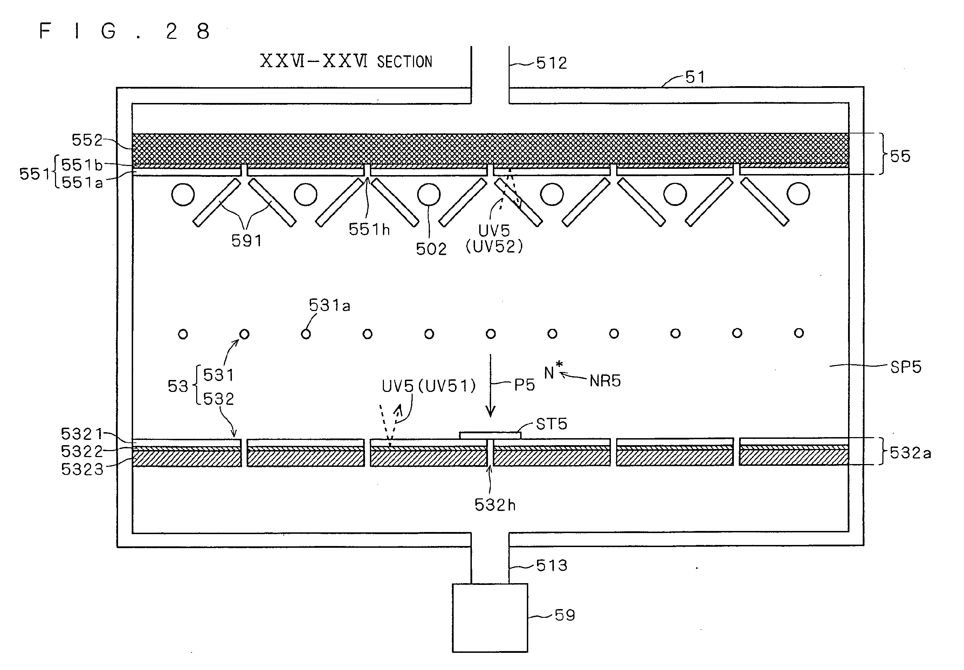

FIG. 28 is a cross-sectional view schematically showing the configuration of a reactor 51. FIG. 28 is a cross-sectional view showing cross-section of the reactor 51 at XXVI-XXVI section in FIG. 27.

As shown in FIG. 28, the electrode pair 53 connected to the pulse power supply 54 is disposed in a space inside the reactor 51 (hereinafter referred to as "aseptization space") where aseptization of the aseptization object substance ST5 is performed.

An anode 531 constituting the electrode pair 53 is configured in such that a plurality of electrode bars 531a having diameter of 0.5 mm are disposed horizontally at a constant interval, and a cathode 532 constituting the electrode pair 53 is configured in such that one sheet of electrode plate 532a is disposed horizontally.

In the reactor 51, for materials of the anode 531, INCONEL (registered trademark) having excellent durability is employed. However, this does not discouraged that for materials of the anode 531, those other than INCONEL (registered trademark), for example, those composed primarily of metals such as tungsten, molybdenum, manganese, titanium, chrome, zirconium, nickel, silver, iron, copper, platinum and palladium are used. Of course, as the anode 531, use of an electrode bar onto which a metallic film composed primarily of these metals or nitride film is formed using film forming means such as plating, CVD or the like is not discouraged. Meanwhile, "metal" as used herein includes an alloy containing two or more kinds of metals such as nickel alloy or iron alloy as typically represented by stainless steel.

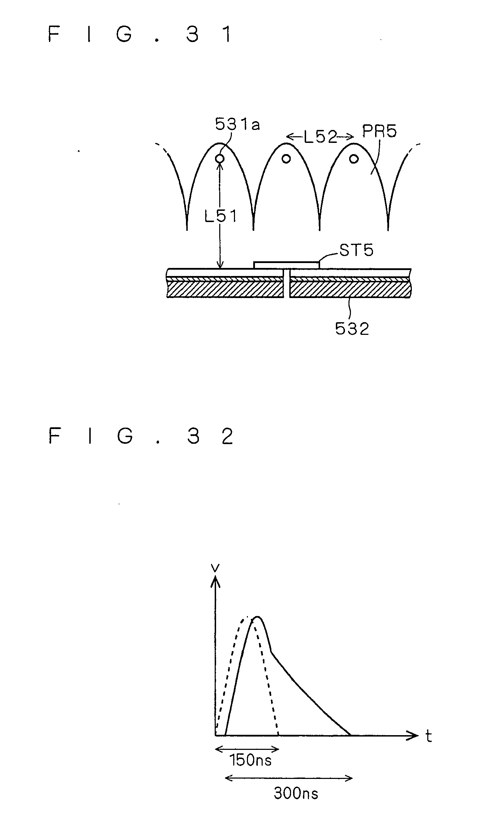

A spacing L52 of the electrode bar 531a is determined with regard to a relationship between spacing L51 of the electrode pair 53 and height of the aseptization object substance ST5. This is because, as shown in FIG. 31, the plasma PR5 required for aseptization is generated around the anode 531a and is widened towards the cathode 532, while its width is widened towards the end as coming closer to the cathode plate 532a, and therefore, in order to expose the whole aseptization object substance ST5 to the plasma PR5, a relationship between the spacing L51 and the spacing L52 is naturally limited. Meanwhile, FIG. 31 is a drawing showing plasma PR5 generation state.

Although it is not discouraged that the anode 531 is configured of electrode plate, in this case, in order to prevent that the anode 531 blocks a short-wavelength ultraviolet ray UV5 and disturbs irradiation of the short-wavelength ultraviolet ray UV5 to the aseptization object substance ST5, the electrode plate in mesh shape or comb teeth shape is desirably adopted so that opposite side may be seen through.

The cathode 532 is configured in such that an aluminum film 5322 with thickness of 0.5 to 10.0 $\mu$m is film formed by vapor deposition onto one principal surface of a quartz glass plate 5321 with thickness of 0.5 to 5.0 mm. In the cathode 532, another principal surface, onto which the aluminum film 5322 is not film formed, of the quartz glass plate 5321 is directed towards the aseptization object substance ST5.

In the cathode 532, in order to ensure electric continuity, the aluminum film 5322 is closely contacted to a metallic electrode 5323 connected to the pulse power supply 54.

To the cathode 532 is formed a ventilating hole 532h to allow nitrogen gas flow from upper part where the intake port 512 is provided towards lower part where the exhaust port 513 is provided.

In the reactor 51, the aseptization object substance ST5 is placed directly on the cathode plate 532a.

In the cathode 532, since the aluminum film 5322 is covered with the quartz glass plate 5321 that functions as a dielectric body barrier, when the electric pulse is applied, a time before the electric current I is discontinued - becomes longer. For this reason, input electric power I x V increases in the reactor 51, temperature of nitrogen atmosphere increases, and aseptization can be performed effectively. For example, as shown by rough waveforms in FIG. 32, even if pulse width $\Delta$t of the electric pulse is 150 ns for a case where the cathode 532 not covered with the quartz glass plate 5321 is used, if the cathode 532 covered with the quartz glass plate 5321 is used, the electric pulse exhibits a long skirt (tailing), pulse width $\Delta$t is lengthened up to about 300 ns, and input electric power increases.

Meantime, FIG. 32 is a graph showing changes in voltage V (vertical axis) with regard to time t (horizontal axis), where dotted line shows rough waveform of the electric pulse when the cathode 532 not covered with the quartz glass plate 5321 is used, solid line shows rough waveform of the electric pulse when the cathode 532 covered with the quartz glass plate 5321 is used.

Since elongation of a skirt of the electric pulse is depending on electrostatic capacitance across the electrode pair 53, thickness of the quartz glass is desirably determined so that the input electric power is appropriate.

With the configuration as mentioned, in the reactor 51 in which the aseptization space SP5 is filled with nitrogen atmosphere, when an electric pulse is applied to the electrode pair 53 in a state where the aseptization object substance ST5 is present between the electrode pair 53, (1) pulse electric field P5 generated by electric pulse application to the electrode pair 53, (2) nitrogen radical NR5 contained in the plasma generated in nitrogen atmosphere resulting from fine streamer discharge, and (3) short-wavelength ultraviolet ray UV5 emitted by nitrogen atmosphere resulting from fine streamer discharge do act on bacteria adhered onto the aseptization object substance ST5, and the aseptization object substance ST5 is aseptized.

Further, in the reactor 51, fine streamer discharge is induced in the nitrogen atmosphere pressure reduced and made lean down to about 1/10 to 1/2 of the atmospheric pressure, in order to lengthen the discharge distance and to secure the spacing L51 of the electrode pair 53 (typically, more than five times that of atmospheric pressure case) that enables aseptization of three-dimensional aseptization object substance ST5. In addition, inducing fine streamer discharge in the pressure reduced and made lean nitrogen atmosphere contributes to lengthening of lifetime of nitrogen radical NR5 (typically more than 10 times that of atmospheric pressure case) and to effective aseptization of the aseptization object substance ST5.

Further, in the reactor 51, nitrogen gas flow parallel to the pulse electric field P5 is created so as to allow further lengthening of the spacing L51 of the electrode pair 53. With this consideration, in the aseptization apparatus 5, arc discharge occurs hardly even if the aseptization object substance ST5 is a metal, especially an article having a sharp-edged portion such as pincette. Furthermore, creation of a nitrogen gas flow parallel to the pulse electric field P5 enables improvement of uniformity of plasma generation and aseptization, and there is such an advantage that generation of ozone, which disturbs aseptization, can be suppressed to a level which does not pose a practical problem, even if the reactor 51 is not sealed completely. This is attributable to that if a nitrogen gas flow flowing from the anode 531 parallel to the pulse electric field towards the cathode 532 is created, oxygen gas is hardly mixed thereto. Meanwhile, since emission of pink color light is observed when ozone is generated, presence or absence of ozone generation can be confirmed easily.

The cathode 532 provided to inner surface at lower part of the reactor 51 also serves as a mirror for reflecting short-wavelength ultraviolet ray UV5 and functions such that a short-wavelength ultraviolet ray UV51 escaping from inside of the aseptization space SP5 to lower part is reflected and is returned to inside of the aseptization space SP5. In the reactor 51, utilization efficiency of the short-wavelength ultraviolet ray UV5 emitted by the nitrogen atmosphere is improved by providing such cathode 532 and improvement of aseptization efficiency is attempted by increasing the short-wavelength ultraviolet ray UV5 acting on the bacteria adhered onto the aseptization object substance ST5. Here, formation of a mirror finished surface by the aluminum film 5322 is due to the fact that, as explained in the first embodiment, reflection coefficient of short-wavelength ultraviolet ray UV5 of the aluminum film is extremely high (about 90%). Meanwhile, from the fact that if the aluminum film 5232 is deteriorated, aseptization efficiency is greatly reduced, contribution of the short-wavelength ultraviolet ray UV5 to aseptization is apparent.

Further, to upper part of the reactor 51 is pasted a reflecting plate 55 which reflects a short-wavelength ultraviolet ray UV52 escaping from inside of the aseptization apparatus SP5 to upper part and returns it to inside of the aseptization apparatus SP5.

As shown in FIG. 28, the reflecting plate 55 is configured in such that a laminated mesh 552 made of stainless steel is placed and fixed on a hole drilling mirror 551 being film formed by vapor deposition of an aluminum film 551b on upper face of a glass plate 551a. In the reflecting plate 55, lower face of glass plate 551a on which the aluminum film 551b is not formed is directed towards the aseptization object substance ST5. In the reactor 51, utilization efficiency of the short-wavelength ultraviolet ray UV5 emitted by the nitrogen atmosphere is improved by providing the reflecting plate 55, and improvement of aseptization efficiency is attempted by increasing the short-wavelength ultraviolet ray UV5 acting on the bacteria adhered onto the aseptization object substance ST5.

Here, formation of a mirror finished surface by the aluminum film 551b is due to the fact that, similar to the case of the cathode 532, reflection coefficient of short-wavelength ultraviolet ray of the aluminum film is extremely high (about 90%).

To the hole drilling mirror 551 are formed penetration holes 551h penetrating the upper face and the lower face regularly in both longitudinal and lateral directions. The hole drilling mirror 551 can be manufactured similar to the hole drilling mirror 351 in the third embodiment.

In the reactor 51, nitrogen gas supplied via the intake port 512 is injected towards the aseptization object substance ST5 through opening of the mesh 552 and the penetration holes 551h of the hole drilling mirror 551 to convert atmosphere of the space SP5 where the aseptization object substance ST5 is present to nitrogen atmosphere. Here, since aseptization space SP5 is pressure-reduced and mean free path of nitrogen molecules is lengthened, the nitrogen gas being injected from the penetration holes 551h is diffused from the penetration holes 551h in a radial pattern. Thus, in the reactor 51, the nitrogen gas can be injected efficiently to the aseptization object substance ST5.

The mesh 552 as a mesh spacing of about 0.1 mm and functions as a buffer for uniforming injection of the nitrogen gas from a number of penetration holes 551h. Adoption of such a buffer can prevent that injection of the nitrogen gas is concentrated to the penetration holes 551h in the vicinity of the intake port thereby disturbing uniformity of aseptization. Use of the mesh 552 as a pressure drop member of the nitrogen gas is not essential, and madreporite of ceramics such as alumina and SiC may be used.

A gas flow guide 591 functions as a guide of nitrogen gas to ensure radial diffusion of nitrogen gas. In order to avoid attenuation of short-wavelength ultraviolet ray UV5, the gas flow guide is composed of raw materials such as glass that allows permeation of the short-wavelength ultraviolet ray UV5.

Creation of a nitrogen gas flow as mentioned above can prevent reduction in aseptization efficiency and stability due to accumulation of nitrogen gas.

In the reactor 51, in order to bring nitrogen atmosphere temperature to a temperature most suited for aseptization, temperature of the nitrogen atmosphere is desirably regulated using a heater 502. As the heater 502, an infrared ray heater such as halogen heater or ceramics heater in bar-shape may be used. Here, due to pressure reduction in the aseptization space SP5, temperature of the nitrogen atmosphere should be determined paying attention to that the temperature most suited for aseptization is lower than that of atmospheric pressure case (e.g., for stearothermophilus case, reduction from about 58˚C for atmospheric pressure case to 30˚C to 50˚C). In FIG. 28, although the heater 502 is disposed immediately beneath the reflecting plate 55, in order that the heater 502 does not disturb nitrogen gas injection from the penetration holes 551h, the heater 502 is disposed so as not to block the penetration holes 551h looking from lower part.

Meanwhile, for creation of nitrogen gas flow moving from upper part towards lower part, it is desirable that total hole area S1 of the intake port 512, total hole area S2 of the cathode plate 532a, and total hole area S3 of the exhaust port 513 are in a relationship of S1<S2<S3.

<2.5.1.3 Configuration of pulse power supply>

Waveform of the electric pulse applied repeatedly by the pulse power source 54 to the electrode 53 is determined so that cell membrane of bacteria can be destroyed by the pulse electric field and at the same time, fine streamer is induced without inducing arc discharge, and plasma can be generated stably in the space SP5.

The pulse power source 54 desirably employs the IES circuit 140 explained in the first embodiment.

<2.5.2 Operations of aseptization apparatus>

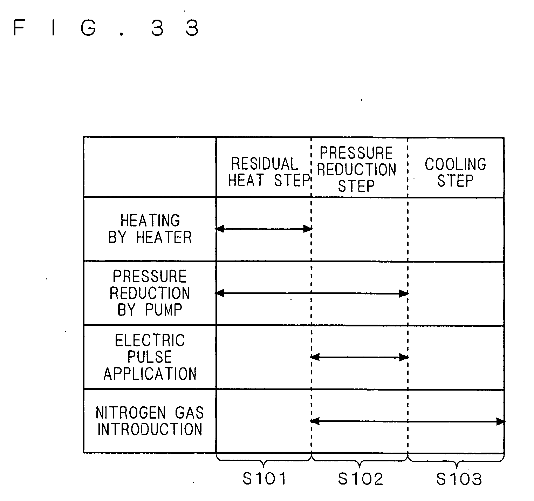

FIG. 33 is a drawing for explanation of operations of the aseptization apparatus 5. FIG. 33 shows presence or absence of heating by the heater 502, presence or absence of pressure reduction by the pump 59, presence or absence of electric pulse to the electrode pair 53, and presence or absence of nitrogen gas introduction for each of a residual heat step, an aseptization step, and a cooling step.

When sealed door 511 is opened, the aseptization object substance ST5 is placed on the cathode plate 532a, and the sealed door 511 is closed again, the aseptization apparatus 5 starts the residual heat step S101. In the residual heat step S101, heating by the heater 502 and pressure reduction by the pump 59 are performed, and inside temperature of the reactor 51 is heated to 40˚C to 60˚C in about 2 minutes.

In the subsequent aseptization step S102, heating by the heater 502 is stopped, and application of an electric pulse to the electrode pair 53 and introduction of nitrogen gas are started while pressure reduction by the pump 59 is continued. The reason for why heating by the heater 502 is stopped in the aseptization step S102 is due to the fact that even if heating by the heater 502 is stopped, inside temperature of the reactor 51 could be maintained thanks to increase in the input electric power resulting from tailing of the electric pulse mentioned previously. The reason why pressure reduction by the pump 59 is continued is to maintain the aseptization space SP5 in a pressured reduced state and to generate a nitrogen gas flow parallel to the pulse electric field P5. In the aseptization step S102, a relatively small amount of nitrogen gas (e.g., 10 liter/min) is introduced in the reactor 51, the pulse electric field P5, short-wavelength ultraviolet ray UV5, and nitrogen radical NR5 do act on bacteria adhered on the aseptization object substance ST5 on the aseptization space SP5, and aseptization of the aseptization object substance ST5 is carried out in about 5 minutes.

In the cooling step S103 that follows, application of electric pulse to the electrode pair 53 and pressure reduction by the pump 59 are stopped, while introduction of the nitrogen gas is continued. In the cooling step S103, a relatively large amount of nitrogen gas (e.g., 20 liter/min) is introduced in the reactor 51, and inside of the reactor 51 is cooled down to room temperature in about 2 minutes. By this cooling step S103, the aseptization object substance ST5 which completed aseptization can be now taken out safely from the reactor 51.

<2.5.3 Variants>

Although the pump 59 is provided outside the reactor 51 in the fifth embodiment, it may be used that the pump 59 is provided inside the reactor 51 so as to convey vibrations of the pump 59 to the aseptization object substance ST5. With this, nitrogen radical NR5 and short-wavelength ultraviolet ray UV5 can act easily on a portion where the aseptization object substance ST5 and electrode plate 532a contact each other, thereby aseptizing the aseptization object substance ST5 more effectively.

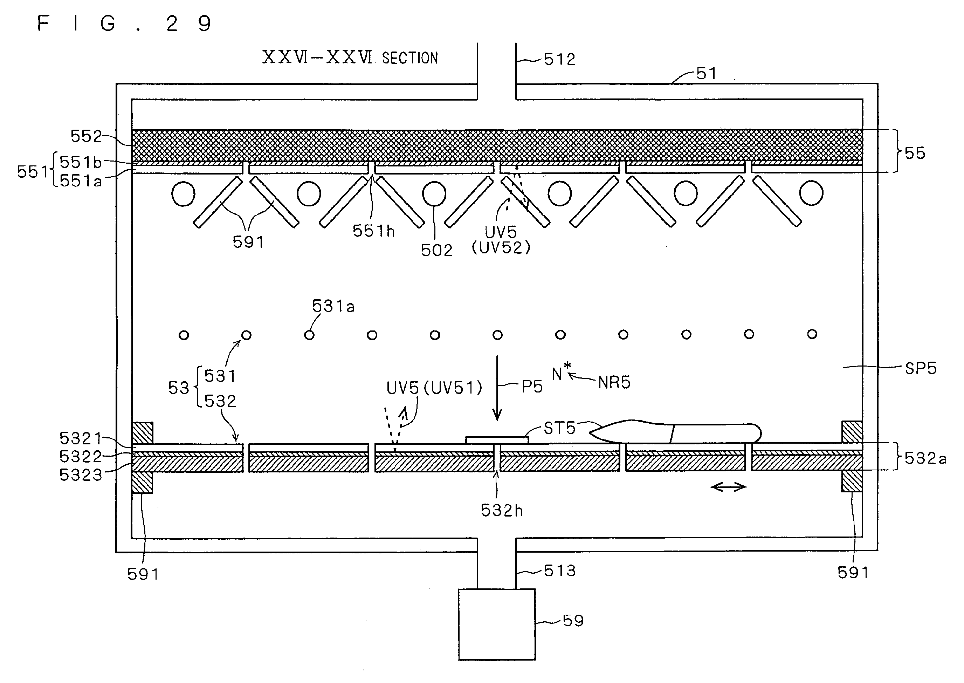

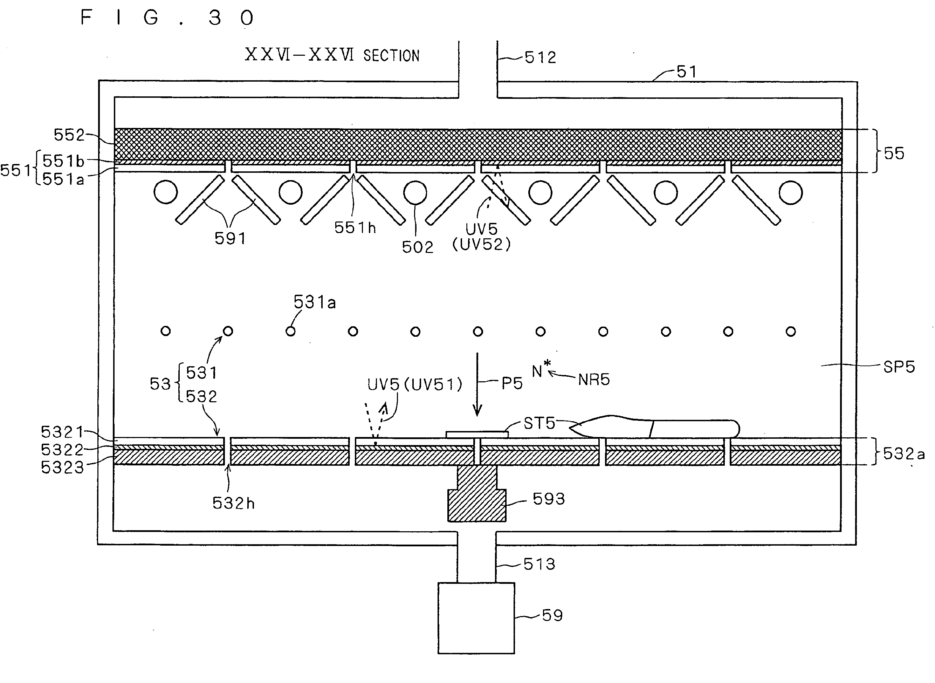

Further, as exemplified in another example shown in FIG. 29 and FIG. 30, a driving mechanism 591 (FIG. 29) for moving the cathode 532 in vertical direction with regard to the pulse electric field P5, or a driving mechanism 593 (FIG. 30) for turning the cathode 532 in vertical direction with regard to the pulse electric field P5 may be provided. This is because such mechanisms are able to uniformize aseptization effects. Adoption of such mechanisms is also effective for the aseptization apparatuses 1 to 4 in the first embodiment to fourth embodiment.

<3. Examples>

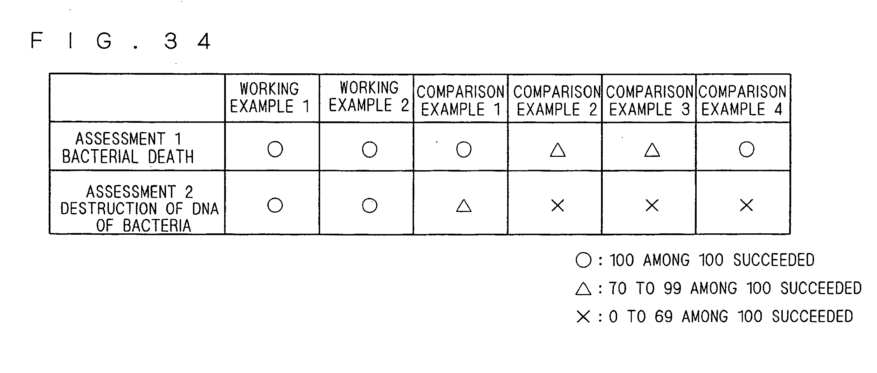

The following description explains, referring to FIG. 34, comparison of aseptization between those attained by the aseptization apparatus 1 according to the first embodiment and by the aseptization apparatus 2 according to the second embodiment, and those attained by the aseptization apparatus outside the scope of the present invention. FIG. 34 is a drawing showing results of experimental assessment of success rate of bacterial death performed using 100 pieces of biological indicators (assessment 1), and results of experimental assessment of success rate of destroying DNA of bacteria performed using 100 pieces of biological indicators (assessment 2).

In the assessment 1 shown in FIG. 34, mark "○" indicates that the bacteria were killed in all of the biological indicators, mark "Δ" indicates that the bacteria were killed in over 70 to 99 or less biological indicators, and mark "×" indicates that the bacteria were killed in only 69 or less biological indicators.

In the assessment 2 show in FIG. 34, mark "○" indicates that DNA of the bacteria could be destroyed in all of the biological indicators, mark "Δ" indicates that DNA of the bacteria could be destroyed in over 70 to 99 or less biological indicators, and mark "×" indicates that DNA of the bacteria could be killed in only 69 or less biological indicators.

In the assessment 1 and assessment 2, biological indicators having bacterial strain of Bacillus (Stearothermophilus) and bacterial volume of $1 \times 10^6$ pieces were used. Judgment of success and failure of bacterial death were assessed by presence or absence of discoloration after the biological indicator was immersed into a culture solution containing pH indicator for 48 hours, and success and failure of destruction of DNA of the bacteria were assessed by observation of the biological indicator under microscope. In the examples 1 to 2, and comparison examples 1 to 4, input energy was assumed to be constant (200 J) throughout the assessment.

(Example 1)

Aseptization was carried out using the aseptization apparatus 1 of the first embodiment and results equivalent to mark "○" were obtained in both assessment 1 and assessment 2.

(Example 2)

Aseptization was carried out using the aseptization apparatus 2 of the second embodiment and results equivalent to mark "○" were obtained in both assessment 1 and assessment 2, similar to Example 1.

(Comparison example 1)

In the aseptization apparatus 1 of the first embodiment, the mirror 15 was removed, inner surface of the sealed container 11 was painted black, and aseptization was carried out under the same conditions as in Example 1. Results equivalent to mark "○" were obtained in assessment 1 while results obtained in assessment 2 were only equivalent to mark "Δ".

(Comparison example 2)

Aseptization was carried out in the aseptization apparatus 1 of the first embodiment under the same conditions as in Example 1 while a mixed gas of helium and neon (mixing ratio; helium : neon =1:1) was supplied to the aseptization space 191 instead of supplying the nitrogen gas to the aseptization space 191, and atmosphere pressure was maintained at less than one atmospheric pressure. Results obtained in assessment 1 were only equivalent to mark "Δ" and results obtained in assessment 2 were only equivalent to mark "×",

(Comparison example 3)

Aseptization was carried out in the aseptization apparatus 1 of the first embodiment under the same conditions as in Example 1 while oxygen gas was supplied to the aseptization space 191 instead of supplying the nitrogen gas to the aseptization space 191. Results obtained in assessment 1 were only equivalent to mark "Δ" and results obtained in assessment 2 were only equivalent to mark "×".

(Comparison example 4)

Aseptization was carried out by irradiating short-wavelength ultraviolet ray to the biological indicators using an ultraviolet lamp. Although results equivalent to mark "○" were obtained in assessment 1, results obtained in assessment 2 were only equivalent to mark "×".

As it is apparent from above-shown working examples 1 to 2, with the aseptization apparatus 1 of the first embodiment and the aseptization apparatus 2 of the second embodiment, aseptization could be performed with a small amount of energy by causing the short-wavelength ultraviolet ray having high bactericidal effects to act effectively on the bacteria, in addition to destroying cell membrane of the bacteria by pulse electric field and destroying DNA of the bacteria by nitrogen radical.

In the meantime, if the short-wavelength ultraviolet ray having high bactericidal effects was not radiated sufficiently to the biological indicators which were aseptization object substance as observed in comparison example 1, thorough destruction of DNA with a small amount of energy was not possible.

Further, as shown in comparison examples 2 to 3, if nitrogen atmosphere was not selected and action of nitrogen radical to the bacteria was not used, the bacteria could not be killed thoroughly with a small amount of energy, and DNA of the bacteria could not be destroyed thoroughly.

Further, as shown in comparison example 4, although the bacteria was killed thoroughly by short-wavelength ultraviolet ray alone, DNA of the bacteria could not be destroyed thoroughly.

**Claims**

1. A sterilization/aseptization apparatus for sterilizing or aseptizing an object substance, comprising:

    an atmosphere control means for converting atmosphere of a space where sterilization or aseptization is performed into nitrogen atmosphere;
    an electrode pair disposed in said space;
    a pulse power supply for repeatedly applying an electric pulse, which induces a fine streamer discharge without inducing an arc discharge, to said electrode pair;
    a reflection member for returning short-wavelength ultraviolet ray going from inside of said space to outside to inside of said space;

    wherein said object substance is sterilized or aseptized by causing a pulse electric field generated by application of the electric pulse to said electrode pair, nitrogen radical contained in plasma generated in the nitrogen atmosphere resulting from the fine streamer discharge, and short-wavelength ultraviolet ray emitted by the nitrogen atmosphere resulting from the fine streamer discharge to act on bacteria.

2. The sterilization/aseptization apparatus according to Claim 1, wherein pulse width of said electric pulse is 50 to 300 ns in terms of half bandwidth.

3. The sterilization/aseptization apparatus according to Claim 1 or Claim 2, wherein said atmosphere control means generates a nitrogen gas flow parallel to said pulse electric field.

4. The sterilization/aseptization apparatus according to any one of Claim 1 through Claim 3, wherein
    said object substance is a sheet;
    said atmosphere control means generates a nitrogen gas flow vertical to said sheet by injecting a nitrogen gas towards said sheet;
    said electrode pair are opposed each other across said sheet; and
    said sheet is sterilized or aseptized by causing said pulse electric field, said nitrogen radical, and said short-wavelength ultraviolet ray to act on bacteria adhered to said sheet.

5. The sterilization/aseptization apparatus according to Claim 4, further comprising a carrying means for causing said sheet to.travel along with a predetermined carrying route.

6. The sterilization/aseptization apparatus according to any of Claim 1 through Claim 3, wherein
    said object substance is a steric substance;
    the apparatus further comprises a pressure reducing means for pressure reducing said space; and
    said sheet is sterilized or aseptized by causing said pulse electric field, said nitrogen radical, and said short-wavelength ultraviolet ray to act on the bacteria adhered to said sheet.

7. The sterilization/aseptization apparatus according to Claim 6, further comprising a rotary mechanism for causing said steric substance to rotate around a rotating shaft being vertical to said pulse electric field.

8. The sterilization/aseptization apparatus according to Claim 6, further comprising a rotary mechanism for causing an anode and a cathode of said electrode pair to rotate around a rotating shaft being vertical to said pulse electric field and to revolve around said steric substance with the same cycle.

9. The sterilization/aseptization apparatus according to any one of Claim 6 through Claim 8, wherein a distance between said electrode pair is not less than 1.2 times of length in the longest direction of said object substance.

10. The sterilization/aseptization apparatus according to any one of Claim 6 through Claim 9, further comprising a holding means for holding an aseptization object inside said space, wherein at least a part of said holding means is in mesh shape or comb teeth shape.

11. The sterilization/aseptization apparatus according to Claim 10, wherein material of said holding means is iron alloy or tungsten alloy.

12. The sterilization/aseptization apparatus according to any one of Claim 6 through Claim 11, further comprising a turbulence generation means for generating a turbulence in the nitrogen atmosphere and exposing said object substance to the turbulence.

13. The sterilization/aseptization apparatus according to any one of Claim 1 through Claim 12, wherein said reflection member has a reflection coefficient of said short-wavelength ultraviolet ray not less than 30%.

14. The sterilization/aseptization apparatus according to Claim 13, wherein said reflection member reflects said short-wavelength ultraviolet ray by an aluminum film.

15. The sterilization/aseptization apparatus according to Claim 14, wherein said aluminum film is covered with silicon oxide film or magnesium fluoride film.

16. The sterilization/aseptization apparatus according to any one of Claim 1 through Claim 15, further comprising a temperature regulating means for regulating a temperature of said nitrogen atmosphere.

17. The sterilization/aseptization apparatus according to any one of Claim 1 through Claim 16, wherein at least one of anode and cathode of said electrode pair is in mesh shape or comb teeth shape.

18. The sterilization/aseptization apparatus according to any one of Claim 1 through Claim 17, wherein material of at least one of anode and cathode of said electrode pair is nickel alloy or iron alloy.

19. The sterilization/aseptization apparatus according to any one of Claim 1 through Claim 18, wherein a part of or whole anode and cathode of said electrode pair is covered with an insulating material.

20. The aseptization apparatus according to any one of Claim 1 through Claim 19, wherein anode of said electrode pair is a cylindrical-shape electrode having a diameter of not less than 0.3 mm and not more than 3.0 mm.

21. The sterilization/aseptization apparatus according to any one of Claim 1 through Claim 20, wherein cathode of said electrode pair comprises a quartz glass plate and an aluminum film formed on one principal surface of said quartz glass plate, and another principal surface of said quartz glass plate is faced towards said object substance.

22. The sterilization/aseptization apparatus according to any one of Claim 1 through Claim 21, wherein said atmosphere control means comprises a plate-shape member to which a penetration hole penetrating through both principal surfaces is formed, and wherein a nitrogen gas is injected through said penetration hole.

23. The sterilization/aseptization apparatus according to Claim 22, wherein a plurality of first grooves extending in a first direction are excavated to one principal surface of said plate-shape member and at the same time, a plurality of second groves extending in a second direction, being different from said first direction, are excavated to another principal surface of said plate-shape member, and wherein an intersection of said first grooves and said second grooves serves as said penetration hole.

24. The sterilization/aseptization apparatus according to Claim 22 or Claim 23, wherein said atmosphere control means further comprises a mesh being laminated onto one principal surface of said plate-shape member, and the nitrogen gas is injected passing sequentially through openings of said mesh and said penetration hole of said plate-shape member.

25. The sterilization/aseptization apparatus according to Claim 22 or Claim 23, wherein said atmosphere control means further comprises a ceramic madreporite on one principal surface of said plate-shape member, and the nitrogen gas is injected passing sequentially through openings of said madreporite and said penetration hole of said plate-shape member.

26. The sterilization/aseptization apparatus according to any one of Claim 1 through Claim 25, further comprising a mechanism for moving or turning cathode of said electrode pair in vertical direction with regard to said pulse electric field.

F I G . 1

F I G . 2

F I G . 3

FIG.4

| GLOW DISCHARGE | FINE STREAMER DISCHARGE | STREAMER DISCHARGE | ARC DISCHARGE |
|---|---|---|---|

VOLTAGE APPLIED BEFORE DISCHARGE

$\Delta t > 100ns$

$\Delta t = 100 \sim 400ns$

$\Delta t = 500 \sim 1000ns$

$\Delta t > 1000ns$

EP 1 941 912 A1

FIG. 6

EP 1 941 912 A1

EP 1 941 912 A1

F I G . 7

F I G . 8

FIG. 9

EP 1 941 912 A1

FIG. 10

EP 1 941 912 A1

FIG. 11

2

21

251
25 { 252
253

22

NITROGEN GAS SUPPLYING SYSTEM

28

ST2

26

251
252 } 25
253

28

UV2

23(231)   23(231)   23(231)

N*   N*   PR2

253
25 { 252
251

23(232)

295

SP2

26

253
25 { 252
251

253
252 } 25
251

Y
Z ⊙ →X

PULSE POWER SUPPLY

24

EP 1 941 912 A1

F I G . 1 2

F I G . 1 3

F I G . 1 4

EP 1 941 912 A1

31   P31   ENLARGED VIEW IN FIG. 14   SP3   35   ST3

A

33(331)

312

33(331) P32   38

33(332)

35

38

35

33   38

35

33(332)

33(331)

35

38

35

33(332)

33   38

33(331)

35

38

32

35

33(332)

35

38

33   38

33(331)

35

33(332)

NITROGEN GAS

33   38

33(331)

35

38

311

35

33(332)

P3

Z

Y ← ⊗ X

# F I G . 1 5

ENLARGED VIEW OF A-PORTION IN FIG. 13

# F I G . 1 6

F I G . 1 7

# F I G . 1 8

(A)
t=0～t1

+331a

+ + +
ST3
+ + +

- - -
332a
332 332b

Z
Y ⊗X

(B)
t=t1～t2

331a

+ UV3
+

N* ↓ SD3
+ + + + +
ST3
+ + + + +

↓ SD3

- - -
332a
332 332b

Z
Y ⊗X

F I G . 1 9

(C)
t=t2〜t3

331a

SD3

ST3

SD3

332a
332b

Z

Y ⊗X

(D)
t=t3〜t4

331a

SD3

ST3

SD3

332a
332b

Z

Y ⊗X

F I G . 2 0

EP 1 941 912 A1

F I G . 2 2

FIG. 23

XXI-XXI SECTION

45

EP 1 941 912 A1

# F I G . 2 4

(A)
 t=0～t1

431

P4

ST4

432

(B)
 t=t1～t2

431

UV4

NR4

N*

I  SD4

P4

ST4

I  SD4

432

FIG. 25

(A)
t=t2∼t3

(B)
t=t3∼t4

F I G . 2 6

XX–XX SECTION

49

492

491

422

421

41

499

499

415

415

UV4 (UV42)

UV4 (UV41)

NR4

N*

ST4

P4

431a

431
432
43

432

432a

F I G . 2 7

XXVI–XXVI SECTION

EP 1 941 912 A1

F I G . 2 9

XXVI–XXVI SECTION

F I G . 3 1

F I G . 3 2

F I G . 3 3

| | RESIDUAL HEAT STEP | PRESSURE REDUCTION STEP | COOLING STEP |
|---|---|---|---|
| HEATING BY HEATER | ←——→ | | |
| PRESSURE REDUCTION BY PUMP | ←—————————→ | | |
| ELECTRIC PULSE APPLICATION | | ←———→ | |
| NITROGEN GAS INTRODUCTION | | ←—————————→ | |
| | S101 | S102 | S103 |

# FIG. 30

XXVI—XXVI SECTION

EP 1 941 912 A1

F I G . 3 4

| | WORKING EXAMPLE 1 | WORKING EXAMPLE 2 | COMPARISON EXAMPLE 1 | COMPARISON EXAMPLE 2 | COMPARISON EXAMPLE 3 | COMPARISON EXAMPLE 4 |
|---|---|---|---|---|---|---|
| ASSESSMENT 1 BACTERIAL DEATH | ○ | ○ | ○ | △ | △ | ○ |
| ASSESSMENT 2 DESTRUCTION OF DNA OF BACTERIA | ○ | ○ | △ | × | × | × |

○ : 100 AMONG 100 SUCCEEDED

△ : 70 TO 99 AMONG 100 SUCCEEDED

× : 0 TO 69 AMONG 100 SUCCEEDED

EP 1 941 912 A1

## INTERNATIONAL SEARCH REPORT

| International application No. |
| --- |
| PCT/JP2006/321128 |

A. CLASSIFICATION OF SUBJECT MATTER
*A61L2/02*(2006.01)i, *A61L2/10*(2006.01)i, *A61L2/14*(2006.01)i

According to International Patent Classification (IPC) or to both national classification and IPC

B. FIELDS SEARCHED

Minimum documentation searched (classification system followed by classification symbols)
A61L2/02, A61L2/10, A61L2/14

Documentation searched other than minimum documentation to the extent that such documents are included in the fields searched
Jitsuyo Shinan Koho        1922-1996   Jitsuyo Shinan Toroku Koho   1996-2007
Kokai Jitsuyo Shinan Koho   1971-2007   Toroku Jitsuyo Shinan Koho   1994-2007

Electronic data base consulted during the international search (name of data base and, where practicable, search terms used)

C. DOCUMENTS CONSIDERED TO BE RELEVANT

| Category* | Citation of document, with indication, where appropriate, of the relevant passages | Relevant to claim No. |
| --- | --- | --- |
| Y | JP 2002-263169 A (Nippon Paint Co., Ltd.), 17 September, 2002 (17.09.02), Claims; Figs. 1 to 5 (Family: none) | 1-26 |
| Y | JP 2000-295981 A (Biological Specific Industry Technological Research Propellant Organization), 24 October, 2000 (24.10.00), Claims; Figs. 1 to 3 (Family: none) | 1-26 |
| Y | JP 63-318947 A (Ishikawajima-Harima Heavy Industries Co., Ltd.), 27 December, 1988 (27.12.88), Claim; examples; effect of the invention; Fig. 2, 3 (Family: none) | 1-26 |

☒ Further documents are listed in the continuation of Box C.    ☐ See patent family annex.

| | |
| --- | --- |
| * Special categories of cited documents:<br>"A" document defining the general state of the art which is not considered to be of particular relevance<br>"E" earlier application or patent but published on or after the international filing date<br>"L" document which may throw doubts on priority claim(s) or which is cited to establish the publication date of another citation or other special reason (as specified)<br>"O" document referring to an oral disclosure, use, exhibition or other means<br>"P" document published prior to the international filing date but later than the priority date claimed | "T" later document published after the international filing date or priority date and not in conflict with the application but cited to understand the principle or theory underlying the invention<br>"X" document of particular relevance; the claimed invention cannot be considered novel or cannot be considered to involve an inventive step when the document is taken alone<br>"Y" document of particular relevance; the claimed invention cannot be considered to involve an inventive step when the document is combined with one or more other such documents, such combination being obvious to a person skilled in the art<br>"&" document member of the same patent family |

| Date of the actual completion of the international search | Date of mailing of the international search report |
| --- | --- |
| 18 January, 2007 (18.01.07) | 30 January, 2007 (30.01.07) |

| Name and mailing address of the ISA/<br>Japanese Patent Office | Authorized officer |
| --- | --- |
| Facsimile No. | Telephone No. |

Form PCT/ISA/210 (second sheet) (April 2005)

**INTERNATIONAL SEARCH REPORT**

| International application No. |
|---|
| PCT/JP2006/321128 |

C (Continuation).   DOCUMENTS CONSIDERED TO BE RELEVANT

| Category* | Citation of document, with indication, where appropriate, of the relevant passages | Relevant to claim No. |
|---|---|---|
| Y | JP 2002-151295 A  (Yaskawa Electric Corp.), 24 May, 2002 (24.05.02), Claims; Par. Nos. [0005], [0011]; Figs. 1 to 8 (Family: none) | 1-26 |
| Y | JP 2003-62047 A  (Dainippon Printing Co., Ltd.), 04 March, 2003 (04.03.03), Claims; Par. Nos. [0010], [0025], [0035] to [0036] (Family: none) | 1-26 |
| Y | JP 63-174658 A  (Sumitomo Electric Industries, Ltd.), 19 July, 1988 (19.07.88), Claims 1, 7, 8 & EP 277505 A1          & DE 3870268 A & AU 1019688 A          & CN 88100319 A & CA 1297262 A | 1-26 |
| A | JP 7-303688 A  (Takasago Thermal Engineering Co., Ltd.), 21 November, 1995 (21.11.95), Claims; Par. Nos. [0022] to [0024], [0040] to [0044] (Family: none) | 1-26 |

Form PCT/ISA/210 (continuation of second sheet) (April 2005)